# FASCICULE DE BREVET EUROPEEN

(11) **EP 1 101 770 B1**
(45) Date de publication et mention de la délivrance du brevet: **16.04.2003**
(21) Numéro de dépôt: 00403107.6
(22) Date de dépôt: 09.11.2000
(51) Int. Cl.: C07H 19/23, A61K 31/70, A61P 35/00

(54) **Nouveaux dérives de 12,13-(pyranosyl)-indolo(2,3-a)pyrrolo(3,4-c)carbazole et 12,13-(pyranosyl)-furo(3,4-c)indolo(2,3-a)carbazole, leur procédé de préparation et les compositions pharmaceutiques qui les contiennent**
12,13-(pyranosyl)-indolo(2,3-a)pyrrolo(3,4-c)carbazol- und 12,13-(pyranosyl)-furo(3,4-c)indolo(2,3-a)carbazol- Derivate, ihr Verfahren zur Herstellung und pharmazeutische Zusammensetzungen, diese enthaltend
Derivatives of 12,13-(pyranosyl)-indolo(2,3-a)pyrrolo(3,4-c)carbazole and 12,13-(pyranosyl)-furo(3,4-c)indolo(2,3-a)carbazole, their process of preparation and pharmaceutical compositions containing them

(30) Priorité: 17.11.1999 FR 9914433
(43) Date de publication de la demande: 23.05.2001
(73) Titulaire: LES LABORATOIRES SERVIER, 92200 Neuilly sur Seine (FR)
(72) Inventeur: Prudhomme, Michelle, 63000 Clermont-Ferrand (FR); Moreau, Pascale, 63100 Clermont-Ferrand (FR); Anizon, Fabrice, 63720 Ennezat (FR); Marminon, Christelle, 78600 Maison-Lafitte (FR); Atassi, Ghanem, 92210 Saint-Cloud (FR); Pierre, Alain, 78580 Les Alluets le Roi (FR); Pfeiffer, Bruno, 95320 Saint Leu la Foret (FR); Renard, Pierre, 78150 Le Chesnay (FR)

(56) Documents cités:
- WO-A-99/02532
- F.ANIZON ET AL.: "Syntheses , Biochemical and Biological Evaluation of Staurosporine Analogues from the Microbial Metabolite Rebeccamycin." BIOORGANIC AND MEDICINAL CHEMISTRY, vol. 6, 1998, pages 1598-1604, XP000909178

## Description

La présente invention concerne de nouveaux dérivés de 12,13-(pyranosyl)-indolo[2,3-*a*] pyrrolo[3,4-*c*]carbazole et 12,13-(pyranosyl)-furo[3,4-*c*]indolo[2,3-*a*]carbazole, leur procédé de préparation et les compositions pharmaceutiques qui les contiennent.

Les besoins de la thérapeutique anticancéreuse exigent le développement constant de nouveaux agents cytotoxiques, dans le but d'obtenir à la fois des médicaments plus actifs et mieux tolérés. Les composés de la présente invention présentent notamment des propriétés anti-tumorales, les rendant ainsi utiles dans le traitement des cancers.

Diverses modifications chimiques ont été réalisées sur le squelette de la rebeccamycine ou de la staurosporine, en vue d'en améliorer le potentiel anti-tumoral. On peut citer notamment les demandes de brevets WO 98/07433, WO 99/02532 et EP 602 597 qui revendiquent des dérivés de la rebeccamycine comportant des modifications structurales tant sur la partie osidique de la molécule que sur les substituants présents au niveau du système hexacyclique. De même l'article publié dans « Bioorganic and Medicinal Chemistry 1998, 6, *1597-1604* » décrit de tels dérivés présentant une activité cytotoxique correcte.

Les composés de l'invention, outre le fait qu'ils soient nouveaux, présentent une activité in-vitro et in-vivo surprenante et supérieure à celle observée jusqu'ici. Ainsi, les composés découverts par la Demanderesse possèdent des propriétés anti-tumorales qui les rendent particulièrement utiles pour le traitement des cancers.

Plus particulièrement, la présente invention concerne les composés de formule (I) : 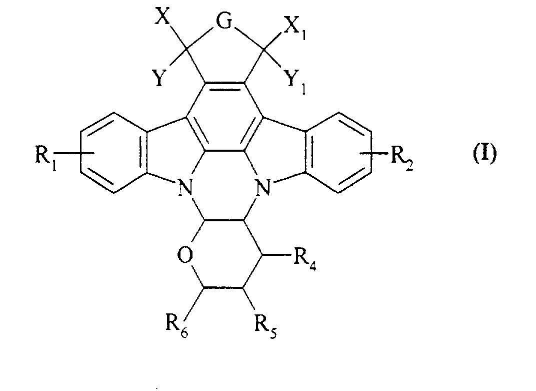 dans laquelle :
- **R**_{**1**}**, R**_{**2**}**,** identiques ou différents, indépendamment l'un de l'autre. représentent un groupement de formule U-V dans laquelle :
   * U représente une liaison simple ou une chaîne alkylène (C₁-C₆) linéaire ou ramifiée, éventuellement substituée par un ou plusieurs groupements, identiques ou différents, choisis parmi halogène et hydroxy, et/ou contenant éventuellement une ou plusieurs insaturations,
   * V représente un groupement choisi parmi atome d'hydrogène, d'halogène, groupement cyano, nitro, azido, alkyle (C₁-C₆) linéaire ou ramifié, aryle, arylalkyle (C₁-C₆) linéaire ou ramifié, hydroxy, alkoxy (C₁-C₆) linéaire ou ramifié, aryloxy, arylalkoxy (C₁-C₆) linéaire ou ramifié, formyle, carboxy, aminocarbonyle, NRaRb, -C(O)-T₁, -C(O)NRa-T₁, -NRa-C(O)-T₁, -O-C(O)-T₁, -C(O)-O-T₁, -O-T₂-NRaRb, -O-T₂-ORa, -O-T₂-CO₂Ra,-NRa-T₂-NRaRb, -NRa-T₂-ORa, -NRa-T₂-CO₂Ra, et -S(O)ₙ-Ra,
   dans lesquels :
   → Ra et Rb, identiques ou différents, représentent chacun un groupement choisi parmi atome d'hydrogène, groupement alkyle (C₁-C₆) linéaire ou ramifié, aryle, et arylalkyle (C₁-C₆) linéaire ou ramifié,
      ou
      Ra+Rb forment ensemble, avec l'atome d'azote qui les portent, un hétérocycle de 5 à 7 chaînons, monocyclique, contenant éventuellement au sein du système cyclique un second hétéroatome choisi parmi oxygène et azote, et étant éventuellement substitué par un groupement choisi parmi alkyle (C₁-C₆) linéaire ou ramifié, hydroxy, alkoxy (C₁-C₆) linéaire ou ramifié, amino, alkylamino (C₁-C₆) linéaire ou ramifié, et dialkylamino (C₁-C₆) linéaire ou ramifié,
   → T₁ représente un groupement choisi parmi alkyle (C₁-C₆) linéaire ou ramifié, aryle, arylalkyle (C₁-C₆) linéaire ou ramifié, et une chaîne alkylène (C₁-C₆) linéaire ou ramifié substituée par un groupement choisi parmi -ORa, -NRaRb, -CO₂Ra, -C(O)Ra et -C(O)NRaRb dans lesquels Ra et Rb sont tels que définis précédemment,
   → T₂ représente une chaîne alkylène (C₁-C₆) linéaire ou ramifiée,
   → n représente un entier compris entre 0 et 2 inclus,
- **G** représente un atome d'oxygène ou un groupement NR₃ dans lequel R₃ représente un groupement choisi parmi atome d'hydrogène, groupement alkyle (C₁-C₆) linéaire ou ramifié, aryle, arylalkyle (C₁-C₆) linéaire ou ramifié, cycloalkyle, cycloalkylalkyle (C₁-C₆) linéaire ou ramifié, -ORa, -NRaRb, -O-T₂-NRaRb, -NRa-T₂-NRaRb, hydroxyalkylamino (C₁-C₆) linéaire ou ramifié, di(hydroxyalkyl)amino (C₁-C₆) linéaire ou ramifié, -C(O)-Ra, -NH-C(O)-Ra, et une chaîne alkylène (C₁-C₆) linéaire ou ramifié, substituée par un ou plusieurs groupements, identiques ou différents, choisis parmi atomes d'halogène, groupements cyano, nitro, -ORa, -NRaRb, -CO₂Ra, -C(O)Ra, hydroxyalkylamino (C₁-C₆) linéaire ou ramifié, di(hydroxyalkyl)amino (C₁-C₆) linéaire ou ramifié, et -C(O)-NHRa, les groupements Ra, Rb et T₂ ayant les mêmes significations que précédemment,
- **X** représente un groupement choisi parmi atome d'hydrogène, groupement hydroxy, alkoxy (C₁-C₆) linéaire ou ramifié, mercapto, et alkylthio (C₁-C₆) linéaire ou ramifié,
- **Y** représente un atome d'hydrogène,
   ou
   **X** et **Y** forment ensemble, avec l'atome de carbone qui les porte, un groupement carbonyle,
- **X**_{**1**} représente un groupement choisis parmi atome d'hydrogène, groupement hydroxy, alkoxy (C₁-C₆) linéaire ou ramifié, mercapto, et alkylthio (C₁-C₆) linéaire ou ramifié,
- **Y**_{**1**} représente un atome d'hydrogène.
   ou
   **X**_{**1**} et **Y**_{**1**} forment ensemble, avec l'atome de carbone qui les porte, un groupement carbonyle,
- **R**_{**4**}**, R**_{**5**}**,** identiques ou différents, indépendamment l'un de l'autre, représentent chacun un groupement choisi parmi atome d'hydrogène, atome d'halogène, groupement hydroxy, alkoxy (C₁-C₆) linéaire ou ramifié, aryloxy, arylalkoxy (C₁-C₆) linéaire ou ramifié, alkyle (C₁-C₆) linéaire ou ramifié, arylalkyle (C₁-C₆) linéaire ou ramifié, aryle, amino (lui-même étant éventuellement substitué par un ou deux groupements, identiques ou différents, choisis parmi alkyle (C₁-C₆) linéaire ou ramifié, aryle, et arylalkyle (C₁-C₆) linéaire ou ramifié), azido, -N=NRa (dans lequel Ra est tel que défini précédemment), et -O-C(O)-Rc
   dans lequel Rc représente un groupement alkyle (C₁-C₆) linéaire ou ramifié (éventuellement substitué par un ou plusieurs groupements choisis parmi halogène, hydroxy, amino, alkylamino (C₁-C₆) linéaire ou ramifié, et dialkylamino (C₁-C₆) linéaire ou ramifié), aryle, arylalkyle (C₁-C₆) linéaire ou ramifié, cycloalkyle, ou hétérocycloalkyle,
- **R**_{**6**} représente un groupement de formule -U₁-R₄ dans laquelle U₁ représente une liaison simple ou un groupement méthylène, et R4 est tel que défini précédemment,
- ou **R**_{**4**}**, R**_{**5**}**, R**_{**6**}**,** pris deux à deux en positions adjacentes ou non adjacentes, forment avec les atomes de carbone qui les portent, un système cyclique de 3 à 6 chaînons, contenant un ou deux atomes d'oxygène, le groupement restant R₄, R₅ ou R₆ n'appartenant pas au dit système cyclique prenant alors l'une quelconque des définitions de R₄, R₅, ou R₆ précisées précédemment,
leurs isomères, ainsi que leurs sels d'addition à un acide ou à une base pharmaceutiquement acceptable,
étant entendu que les composés de formule (I) sont différents des composés suivants :
- le 1,11-dichloro-12,13-[1,2-(4-O-méthyl-β-D-mannopyranosyl)]-6,7,12,13-tétrahydro (5*H*)-indolo[2,3-a]pyrrolo[3,4-c]carbazole-5,7-dione,
- le 12,13-[1,2-(4-O-méthyl-β-D-mannopyranosyl)-6,7,12,13-tétrahydro(5*H*)-indolo[2,3-a] pyrrolo[3,4-c]carbazole-5,7-dione.
- le 12,13-[1,2-(4-O-méthyl-β-D-mannopyranosyl)-6,7,12,13-tétrahydro(5*H*)-indolo[2,3-a] pyrrolo[3,4-c]carbazole-5-one,
- et le 12,13-[1,2-(4-O-méthyl-β-D-mannopyranosyl)-5,6,12,13-tétrahydro(7*H*)-indolo [2,3-a]pyrrolo[3,4-c]-carbazole-7-one,
étant entendu également que par :
- cycloalkyle, on comprend un groupement monocyclique ou bicyclique, saturé ou insaturé mais sans caractère aromatique, comportant de 3 à 10 atomes de carbone, étant éventuellement substitué par un ou plusieurs groupements, identiques ou différents, choisis parmi halogène, alkyle (C₁-C₆) linéaire ou ramifié, trihalogénoalkyle (C₁-C₆) linéaire ou ramifié, hydroxy, alkoxy (C₁-C₆) linéaire ou ramifié. et amino éventuellement substitué par un ou deux groupements alkyle (C₁-C₆) linéaire ou ramifié,
- hétérocycloalkyle, on comprend un groupement cycloalkyle tel que défini précédemment comportant au sein du système cyclique un ou deux hétéroatomes, identiques ou différents, choisis parmi oxygène, azote et soufre,
- aryle, on comprend un groupement phényle, naphtyle, dihydronaphtyle, tétrahydronaphtyle, indényle ou indanyle, chacun de ces groupements étant éventuellement substitué par un ou plusieurs groupements, identiques ou différents, choisis parmi halogène, alkyle (C₁-C₆) linéaire ou ramifié, trihalogénoalkyle (C₁-C₆) linéaire ou ramifié, hydroxy, alkoxy (C₁-C₆) linéaire ou ramifié, et amino éventuellement substitué par un ou deux groupements alkyle (C₁-C₆) linéaire ou ramifié.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

D'une façon avantageuse, le groupement G préféré selon l'invention est le groupement NR₃ dans lequel R₃ est tel que défini dans la formule (I).

Selon une variante avantageuse de l'invention, les composés préférés sont ceux pour lesquels X et Y forment ensemble, avec l'atome de carbone qui les porte, un groupement carbonyle, et X₁ et Y₁ forment ensemble avec l'atome de carbone qui les porte, un groupement carbonyle.

Selon une autre variante avantageuse, les composés préférés de l'invention sont les composés de formule (Ibis) : 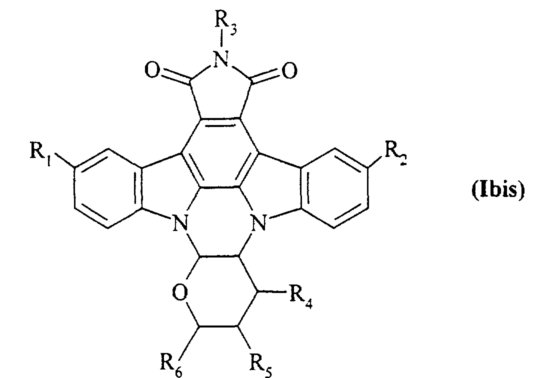 dans laquelle R₁, R₂, R₃, R₄, R₅ et R₆ sont tels que définis dans la formule (I).

D'une façon préférentielle, les substituants R₁ et R₂, selon l'invention, identiques ou différents, représentent un groupement de formule U-V dans laquelle U représente une liaison simple et V représente un groupement choisi parmi atome d'halogène, atome d'hydrogène, groupement nitro, formyle, hydroxy, et une chaîne alkylène (C₁-C₆) linéaire ou ramifiée substituée par un groupement hydroxy.

D'une façon particulièrement avantageuse les substituants R₁ et R₂ sont identiques.

Les substituants R₃ préférés selon l'invention sont l'atome d'hydrogène, le groupement hydroxy et le groupement chaîne alkylène (C₁-C₆) linéaire ou ramifié substituée par un groupement choisi parmi NRaRb et ORa dans lesquels Ra et Rb sont tels que définis dans la formule (I).

Selon une dernière variante particulièrement avantageuse, les composés préférés de l'invention sont les composés de formule (Iter) : 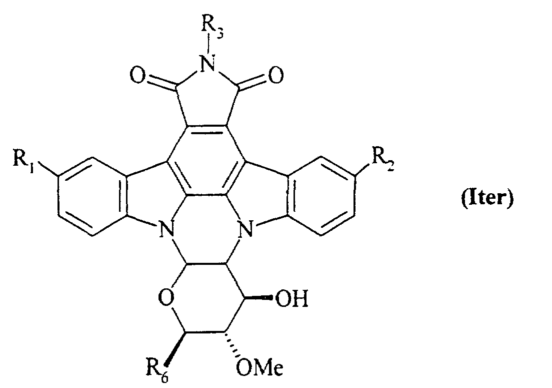 dans laquelle R₁, R₂, R₃ et R₆ sont tels que définis dans la formule (I).

Les composés préférés selon l'invention sont les :
- 3,9-diformyl-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione,
- 3,9-dinitro-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione,
- 3-nitro-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione,
-9-nitro-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione,
-6-hydroxy-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione,
- 3,9-di-(hydroxyméthyl)-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione,
- 3,9-dinitro-12,13-[1,2-(3,6-anhydro-4-*O*-méthyl-β-D-mannopyranosyl)]-6,7,12,13-tétrahydro(5*H*)-indolo[2,3-*a*]pyrrolo[3,4-*c*]carbazole-5,7-dione,
- 3,9-dihydroxy-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione.
- 3,9-dibromo-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione,
- et le 6-diéthylaminoéthyl-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione et son chlorhydrate.

Les isomères ainsi que les sels d'addition à un acide ou à une base pharmaceutiquement acceptable des composés préférés font partie intégrante de l'invention.

La présente invention concerne également le procédé de préparation des composés de formule (I), caractérisé en ce qu'on utilise, comme produit de départ, un composé de formule (II) : 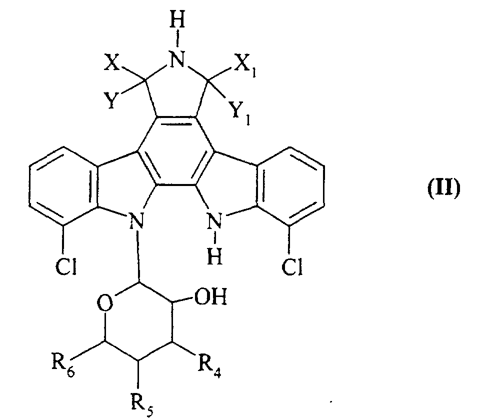 dans laquelle X, Y, X₁, Y₁, R₄, R₅ et R₆ sont tels que définis dans la formule (I), que l'on traite en milieu basique en présence d'acide *para*-toluènesulfonique, pour conduire au composé de formule (III) : 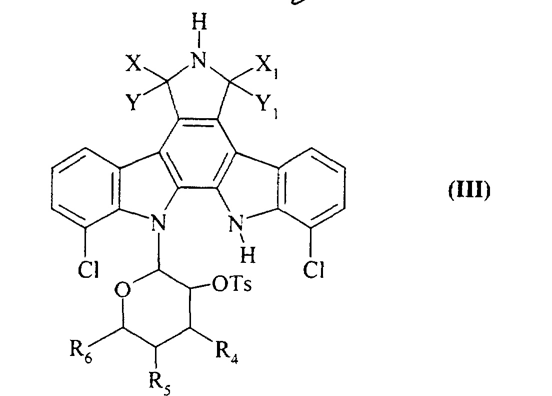 dans laquelle X, Y, X₁, Y₁, R₄, R₅ et R₆ sont tels que définis précédemment, composé de formule (III) qui est mis à réagir avec de l'azoture de sodium, pour conduire principalement au composé de formule (I/a), cas particulier des composés de formule (I) : 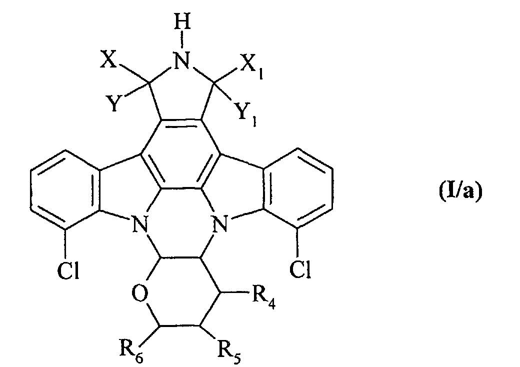 dans laquelle X, Y, X₁, Y₁, R₄, R₅ et R₆ sont tels que définis précédemment, composé de formule (I/a) qui est éventuellement soumis à des conditions d'hydrogénolyse, pour conduire au composé de formule (I/b), cas particulier des composés de formule (I): 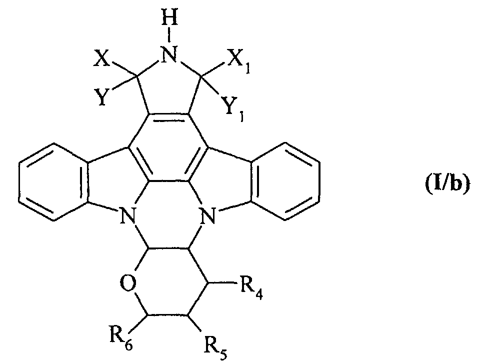 dans laquelle X, Y, X₁, Y₁, R₄, R₅ et R₆ sont tels que définis précédemment, composé de formule (I/b) qui est éventuellement traité par de la soude aqueuse puis placé en présence d'acide chlorhydrique, pour conduire au composé de formule (I/c), cas particulier des composés de formule (I): 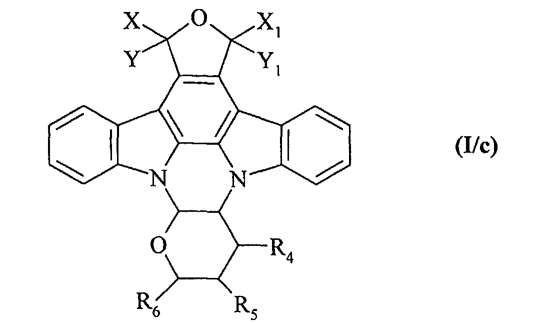 dans laquelle X, Y, X₁, Y₁, R₄, R₅ et R₆ sont tels que définis précédemment, composé de formule (I/c) qui est éventuellement soumis à l'action d'un composé de formule (IV) :

R₃ₐ - NH₂**(IV)**

dans laquelle R₃ₐ a la même définition que R₃ à l'exception de la définition atome d'hydrogène,
pour conduire au composé de formule (I/d), cas particulier des composés de formule (I): 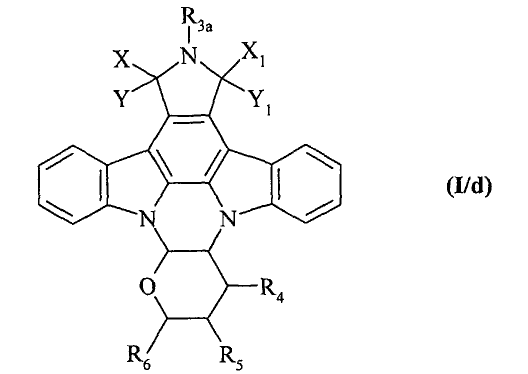 dans laquelle X, Y, X₁, Y₁, R₃ₐ, R₄, R₅ et R₆ sont tels que définis précédemment, l'ensemble des composés de formule (I/b) à (I/d) formant les composés de formule (I/e) : 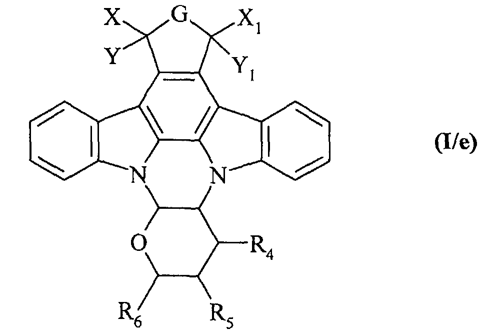 dans laquelle G, X, Y, X₁, Y₁, R₄, R₅ et R₆ sont tels que définis dans la formule (I), composé de formule (I/e) qui est éventuellement soumis à une réaction d'addition électrophile aromatique ou d'addition nucléophile aromatique, selon des conditions classiques de la synthèse organique et bien connues de l'homme de l'art, pour conduire au composé de formule (I/f), cas particulier des composés de formule (I): 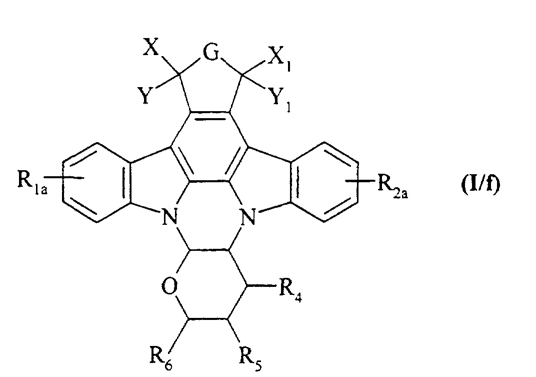 dans laquelle G, X, Y, X₁, Y₁, R₄, R₅ et R₆ sont tels que définis précédemment, et R₁ₐ et R₂ₐ ont les mêmes définitions réciproques que R₁ et R₂ à l'exception que R₁ₐ et R₂ₐ ne peuvent pas représenter simultanément un atome d'hydrogène,
les composés de formule (I/a) à (I/f) formant l'ensemble des composés de formule (I), que l'on purifie, le cas échéant, selon des techniques classiques de purification, qui peuvent, si on le désire, être séparés en leurs différents isomères, selon une technique classique de séparation, dont on module les substituants R₄, R₅ et R₆ selon les méthodes classiques de la synthèse organique utilisées dans le domaine de la chimie des sucres, et que l'on transforme, si on le souhaite, en leurs sels d'addition à un acide ou à une base pharmaceutiquement acceptable.

Les composés de formule (II) et (IV) sont soit des composés commerciaux, soit obtenus selon des méthodes classiques de la synthèse organique facilement accessibles à l'homme du métier.

Les composés de formule (I) présentent des propriétés anti-tumorales particulièrement intéressantes. Ils ont une excellente cytotoxicité in vitro sur des lignées cellulaires, et une action sur le cycle cellulaire. Les propriétés caractéristiques de ces composés permettent leur utilisation en thérapeutique en tant qu'agents antitumoraux.

La présente invention a également pour objet les compositions pharmaceutiques contenant comme principe actif au moins un composé de formule (I), ses isomères optiques, ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse. intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes. les pommades, les gels dermiques, les préparations injectables ou buvables, les aérosols, les gouttes oculaires ou nasales, etc...

De part les propriétés pharmacologiques caractéristiques des composés de formule (I), les compositions pharmaceutiques contenant comme principe actif lesdits composés de formule (I), sont donc particulièrement utiles pour le traitement des cancers.

La posologie utile varie selon l'âge et le poids du patient, la voie d'administration, la nature et la sévérité de l'affection, et la prise de traitements éventuels associés et s'échelonne de 0,5 mg à 500 mg en une ou plusieurs prises par jour.

Les exemples suivant illustrent l'invention mais ne la limitent en aucune façon. Les produits de départs utilisés sont des produits connus ou préparés selon des modes opératoires connus.

Les structures des composés décrits dans les exemples ont été déterminées selon les techniques spectrophotométriques usuelles (infrarouge, résonance magnétique nucléaire, spectrométrie de masse, ...).

### EXEMPLE 1 (exemple de référence) : 1,11-dichloro-12,13-[1,2-(4-O-méthyl-β-D-mannopyranosyl)]-6,7,12,13-tétrahydro(5H)-indolo[2,3-a]pyrrolo[3,4-c]carbazole-5,7-dione

### STADE 1 : 1,11-dichloro-12[4-O-méthyl-2-O-tosyl-β-D-glucopranosyl]-6,7,12,13-tétrahydro(5H)-indolo[2,3-a]pyrrolo[3,4-c]carbazole-5,7-dione

A une solution de 1,7 mmol de rebeccamycine dans 200 ml de tétrahydrofurane sont ajoutés 1 équivalent de carbonate de potassium et 1 équivalent de chlorure de tosyle. Après 48 heures de reflux et concentration sous pression réduite, une chromatographie sur gel de silice (cyclohexane/acétate d'éthyle : 70/30) du résidu permet d'isoler le produit attendu.
**Point de fusion : 168-170°C**

### STADE 2 : 1,11-dichloro-12,13-[1,2-(4-O-méthyl-β-D-mannopyranosyl)]-6,7,12,13-tétrahydro(5H)-indolo[2,3-a]pyrrolo[3,4-c]carbazole-5,7-dione

A une solution de 0,62 mmol du composé obtenu dans le stade 1 dans 16 ml de diméthylformamide sont ajoutés 10 équivalents d'azoture de sodium. Après 6 heures à 70°C, puis refroidissement, le mélange réactionnel est hydrolysé, et extrait à l'acétate d'éthyle. La phase organique est ensuite lavée, séchée, filtrée puis concentrée sous pression réduite. Une chromatographie sur gel de silice (acétate d'éthyle/dichlorométhane : 10/90) permet d'isoler le produit attendu.
**Point de fusion : 296-298°C**
**Spectre de masse (FAB**^{**+**}**): m/z calculé = 551,0650 [M]**^{**+**}
**m/z trouvé = 551,0647 [M]**^{**+**}

### EXEMPLE 2 (exemple de référence) : 12,13-[1,2-(4-O-méthyl-β-D-mannopyranosyl)-6,7,12,13-tétrahydro(5H)-indolo[2,3-a]pyrrolo[3,4-c]carbazole-5,7-dione

Un mélange de 0,855 mmol du composé de l'exemple 1, 0,57 g de Pd/C à 5 % et 0,57 g de formiate d'ammonium dans 200 ml de méthanol est agité à température ambiante pendant 48 heures puis filtré sur célite. Après concentration sous pression réduite du filtrat, une chromatographie sur gel de silice (cyclohexane/acétate d'éthyle : 40/60) permet d'isoler le produit attendu.
**Point de fusion : 284-286°C**
**Spectre de masse (FAB**^{**+**}**) : m/z calculé = 484,1508 [M+H]**^{**+**}
**m/z trouvé = 484,1516 [M+H]**^{**+**}

### EXEMPLE 3 : 12,13-[1,2-(4-O-méthyl-β-D-mannopyranosyl)]-dihydrofuro[3,4-c] indolo[2,3-a]carbazole-5,7-dione

Une solution de 0,414 mmol du composé de l'exemple 2, 420 mg de soude et 70 ml d'eau est portée à reflux pendant 3 heures. Puis le mélange est dilué, acidifié par une solution aqueuse d'acide chlorhydrique 1N et extrait à l'acétate d'éthyle. La phase organique est lavée, séchée, filtrée puis concentrée sous pression réduite. Une chromatographie sur gel de silice (acétate d'éthyle/cyclohexane : 70/30) permet d'isoler le produit attendu.
**Point de fusion : > 300°C**
**Spectre de masse (FAB**^{**+**}**) : m/z calculé = 485,1349 [M+H]**^{**+**}
**m/z trouvé = 485,1333 [M+H]**^{**+**}

### EXEMPLE 4 : 6-méthyl-12,13-[1,2-(4-O-méthyl-β-D-mannopyranosyl)]-6,7,12,13-tétrahydro(5H)-indolo[2,3-a]pyrrolo[3,4-c]carbazole-5,7-dione

0,118 mmol du composé de l'exemple 3 et une solution 2M de méthylamine dans 14 ml de tétrahydrofurane sont agités à 70°C pendant 16 heures. Après refroidissement, le mélange réactionnel est hydrolysé entraînant la formation d'un précipité. Ce dernier est purifié par chromatographie sur gel de silice (acétate d'éthyle/cyclohexane : 80/20) permettant d'isoler le produit attendu.
**Point de fusion : > 300°C**
**Spectre de masse (FAB**^{**+**}**) : m/z calculé = 497,1587 (M)**^{**+**}
**m/z trouvé = 497,1591 (M)**^{**+**}

### EXEMPLE 5 : 6-hydroxy-12,13-[1,2-(4-O-méthyl-β-D-mannopyranosyl)]-6,7,12,13-tétrahydro(5H)-indolo[2,3-a]pyrrolo[3,4-c]carbazole-5,7-dione

A une solution de 0,207 mmol du composé de l'exemple 3 dans 5 ml de diméthylformamide sont ajoutées 14,4 mmol de chlorhydrate d'hydroxylamine, et 14,4 mmol de triéthylamine. Après 23 heures à 70°C, une solution aqueuse d'acide chlorhydrique 1N, de l'acétate d'éthyle et du tétrahydrofurane sont ajoutés. La phase organique est ensuite lavée, séchée, filtrée, puis concentrée sous pression réduite. Une chromatographie sur gel de silice (tétrahydrofurane/méthanol : 95/5) permet d'isoler le produit attendu.
**Point de fusion : > 260°C (décomposition)**

### EXEMPLE 6 : 3,9-diformyl-12,13-[1,2-(3,6-di-O-acétyl-4-O-méthyl-β-D-mannopyranosyl)]-6,7,12,13-tétrahydro(5H)-indolo[2,3-a]pyrrolo [3,4-c]carbazole-5,7-dione

A une solution refroidie à 0°C de 0,207 mmol de composé de l'exemple 2 dans 2 ml de pyridine est ajouté 0,2 ml d'anhydride acétique. Le milieu réactionnel est ensuite agité 18 heures à température ambiante. Après hydrolyse et extraction à l'acétate d'éthyle, la phase organique est lavée, séchée, filtrée puis évaporée. Le résidu obtenu est alors dilué dans 4 ml de dichlorométhane et 4,2 mmol de dichlorométhylméthyléther sont ajoutés. Après refroidissement à 0°C, 4,2 ml d'une solution 1M de TiCl₄ dans le dichlorométhane sont additionnés. Le milieu réactionnel est alors agité 24 heures à température ambiante puis hydrolysé et extrait à l'acétate d'éthyle. La phase organique est séchée, filtrée puis évaporée. Une chromatographie sur gel de silice du résidu (cyclohexane/acétate d'éthyle : 1/1) permet d'isoler le produit attendu.
**InfraRouge (KBr) : ν**_{**C=O**} **= 1690, 1720 et 1755 cm**^{**-1**}
**ν**_{**NH,OH**} **= 3100-3600 cm**^{**-1**}
**Point de fusion : > 200°C**

### EXEMPLE 7 : 3,9-diformyl-12,13-[1,2-(4-O-méthyl-β-D-mannopyranosyl)]-6,7,12,13-tétrahydro(5H)-indolo[2,3-a]pyrrolo[3,4-c]carbazole-5,7-dione

A une solution de 0,074 mmol du composé de l'exemple 6 dans 10 ml de tétrahydrofurane et 5 ml de méthanol sont ajoutés 5 ml d'une solution aqueuse à 28 % d'hydroxyde d'ammonium. Après 30 heures de réaction à 25°C, le milieu est concentré. Le résidu est repris dans du dichlorométhane et lavé à l'eau. La phase organique est évaporée et le résidu obtenu est lavé à l'acétone permettant d'isoler le produit attendu.
**Point de fusion : > 300°C**
**InfraRouge (KBr) : ν**_{**C=O**} **= 1680, 1710 et 1750 cm**^{**-1**}
**ν**_{**NH,OH**} **= 3100-3650 cm**^{**-1**}

### EXEMPLE 8 : 3,9-di(hydroxyméthyl)-12,13-[1,2-(3,6-di-O-acétyl-4-O-méthyl-β-D-mannopyranosyl)]-6,7,12,13-tétrahydro-(5H)-indolo[2,3-a]pyrrolo [3,4-c] carbazole-5,7-dione

Une solution de 0,116 mmol du composé de l'exemple 6, 60 ml de méthanol et 101 mg de Nickel de Raney est hydrogéné à 25°C pendant 2 jours, puis 0,591 g de Nickel de Raney (50 % en masse dans l'eau) est ajouté et l'hydrogénation est maintenue pour 5 jours. Le milieu réactionnel est ensuite filtré sur célite. Après concentration sous pression réduite du filtrat, une chromatographie sur gel de silice (acétate d'éthyle/cyclohexane : 90/10) permet d'isoler le produit attendu.
**Point de fusion : > 180°C (décomposition)**
**InfraRouge (KBr) : ν**_{**C=O**} **= 1720, 1750 cm**^{**-1**}
**ν**_{**NH,OH**} **= 3150-3650 cm**^{**-1**}

### EXEMPLE 9 : 3,9-di-(hydroxyméthyl)-12,13-[1,2-(4-O-méthyl-β-D-mannopyranosyl)]-6,7,12,13-tétrahydro(5H)-indolo[2,3-a]pyrrolo [3,4-c]carbazole-5,7-dione

On procède comme dans l'exemple 7 en utilisant comme substrat le composé de l'exemple 8.
**Point de fusion : > 300°C**
**InfraRouge (KBr) : ν**_{**C=O**} **= 1710, 1750 cm**^{**-1**}
**ν**_{**NH,OH**} **= 3100-3600 cm**^{**-1**}

### EXEMPLE 10 : 12,13-[1,2-(6-chloro-6-déoxy-4-O-méthyl-β-D-mannopyranosyl)]-6,7,12,13-tétrahydro(5H)-indolo[2,3-a]pyrrolo[3,4-c]carbazole-5,7-dione

A une solution de 0,447 mmol du composé de l'exemple 2 dans 2 ml de pyridine sont ajoutés 4 équivalents de PPh₃ et 2 équivalents de CCl₄. Après 3 heures d'agitation à 25°C, le mélange réactionnel est versé dans une solution aqueuse d'acide chlorhydrique 1N puis extrait à l'acétate d'éthyle. La phase organique est lavée, séchée, filtrée puis concentrée sous pression réduite. Une chromatographie sur gel de silice permet d'isoler le produit attendu.
**Point de fusion : > 280°C (décomposition)**
**InfraRouge (KBr) : ν**_{**C=O**} **= 1710, 1750 cm**^{**-1**}
**ν**_{**NH,OH**} **= 3150-3600cm**^{**-1**}

### EXEMPLE 11 : 12,13-[1,2-(3,6-anhydro-4-O-méthyl-β-D-mannopyranosyl)]-6,7,12,13-tétrahydro(5H)-indolo[2,3-a]pyrrolo[3,4-c]carbazole-5,7-dione

Le produit est isolé lors de la chromatographie effectuée dans l'exemple 10.
**Point de fusion : > 300°C**
**InfraRouge (KBr) : ν**_{**C=O**} **= 1720, 1750 cm**^{**-1**}
**ν**_{**NH,OH**} **= 3100-3600 cm**^{**-1**}

### EXEMPLE 12 : 12,13-[1,2-(6-azido-6-déoxy-4-O-méthyl-β-D-mannopyranosyl)]-6,7,12,13-tétrahydro(5H)-indolo[2,3-a]pyrrolo[3,4-c]carbazole-5,7-dione

+Une solution de 0,1 mmol du composé de l'exemple 10 dans 1 ml de diméthylformamide, et 10 équivalents d'azoture de sodium est agitée à 80°C. Après 48 heures, le mélange réactionnel est repris à l'acétate d'éthyle et lavé à l'eau. La phase organique est séchée, filtrée et évaporée. Une chromatographie sur gel de silice (dichlorométhane/acétate d'éthyle : 95/5) permet d'isoler le produit attendu.
**Point de fusion : > 250°C (décomposition)**
**InfraRouge (KBr) : ν**_{**C=O**} **= 1700, 1750 cm**^{**-1**}
**ν**_{**N=N**} **= 2100 cm**^{**-1**}
**ν**_{**NH,OH**} **= 3150-3600 cm**^{**-1**}

### EXEMPLE 13 : 3,9-dinitro-12,13-[1,2-(4-O-méthyl-β-D-mannopyranosyl)]-6,7,12,13-tétrahydro(5H)-indolo[2,3-a]pyrrolo[3,4-c]carbazole-5,7-dione

A une solution de 0,5 mmol du composé de l'exemple 2 dans 4,8 ml de pyridine sont additionnés à 0°C 10 équivalents d'anhydride acétique. Après 1 jour de réaction à 25°C. le milieu réactionnel est hydrolysé, puis extrait à l'acétate d'éthyle. La phase organique est ensuite lavée, séchée, filtrée et évaporée. Le résidu obtenu est dilué dans 10 ml de tétrahydrofurane et 5,6 ml d'acide nitrique fumant sont ajoutés. Après 5 jours à 40°C, 2.8 ml d'acide nitrique fumant sont additionnés et le mélange est maintenu 30 heures à 40°C, puis hydrolysé. Après extraction à l'acétate d'éthyle, la phase organique est lavée, séchée, filtrée, puis concentrée sous pression réduite. Le résidu est alors soumis au procédé décrit dans l'exemple 8 permettant d'isoler le produit attendu.
**Point de fusion : > 300°C**
**InfraRouge (KBr) : ν**_{**C=O**} **= 1690, 1740 cm**^{**-1**}
**ν**_{**NH,OH**} **= 3170-3640 cm**^{**-1**}

### EXEMPLE 14 : 3 et 9-nitro-12,13-[1,2-(4-O-méthyl-β-D-mannopyranosyl)]-6,7,12,13-tétrahydro(5H)-indolo[2,3-a]pyrrolo[3,4-c]carbazole-5,7-dione

On procède comme dans l'exemple 13 en utilisant comme réactif de l'acide nitrique concentré et en réalisant la réaction à température ambiante. La chromatographie permet d'isoler un mélange (1,5/1) de dérivés 3-nitro et 9-nitro.
**Point de fusion du mélange : 293°C**

### EXEMPLE 15 : 3,9-diamino-12,13-[1,2-(4-O-méthyl-β-D-mannopyranosyl)]-6,7,12,13-tétrahydro(5H)-indolo[2,3-a]pyrrolo[3,4-c]carbazole-5,7-dione

Une solution de 0,094 mmol du composé de l'exemple 13 dans 13 ml de tétrahydrofurane et 1,88 mmol de SnCl₂ est mise à reflux pendant 63 heures. Après refroidissement, le milieu réactionnel est hydrolysé et le précipité formé est filtré. Le filtrat est ajusté à pH 10. Après extraction à l'acétate d'éthyle, la phase organique est séchée, filtrée et évaporée permettant d'isoler le produit attendu.
**Point de fusion : > 155°C (décomposition)**
**InfraRouge (KBr) : ν**_{**C=O**} **= 1700, 1710, 1750 cm**^{**-1**}
**ν**_{**NH,OH,NH**^{**2**}} **= 3000-3600 cm**^{**-1**}

### EXEMPLE 16 : 3,9-dinitro-12,13-[1,2-(3,6-anhydro-4-O-méthyl-β-D-mannopyranosyl)]-6,7,12,13-tétrahydro(5H)-indolo[2,3-a] pyrrolo[3,4-c]carbazole-5,7-dione

On procède comme dans l'exemple 13 en utilisant comme substrat le composé de l'exemple 11.
**Point de fusion : > 300°C**
**InfraRouge (KBr) : ν**_{**C=O**} **= 1720, 1760 cm**^{**-1**}
**ν**_{**NH**} **= 3100-3500 cm**^{**-1**}

### EXEMPLE 17 : 3-nitro-12,13-[1,2-(3,6-anhydro-4-O-méthyl-β-D-mannopyranosyl)]-6,7,12,13-tétrahydro(5H)-indolo[2,3-a] pyrrolo[3,4-c]carbazole-5,7-dione

Le produit est isolé lors de la synthèse du composé de l'exemple 16.
**Point de fusion : > 300°C**
**InfraRouge (KBr) : ν**_{**C=O**} **= 1710, 1750 cm**^{**-1**}
**ν**_{**NH**} **= 3200 cm**^{**-1**}

### EXEMPLE 18 : 3,9-dibydroxy-12,13-[1,2-(4-O-méthyl-β-D-mannopyranosyl)] 6,7,12,13-tétrahydro(5H)-indolo[2,3-a]pyrrolo[3,4-c]carbazole-5,7-dione

A une solution de 0,211 mmol du composé de l'exemple 6 dans 6 ml de méthanol sont additionnés 37 µl d'une solution aqueuse à 50 % de H₂O₂ puis 11 µl d'une solution aqueuse d'acide sulfurique à 95 %. Après 72 heures d'agitation à température ambiante, le milieu réactionnel est hydrolysé puis extrait à l'acétate d'éthyle. La phase organique est lavée, séchée, filtrée puis évaporée. Une chromatographie sur gel de silice (toluène/tétrahydrofurane : 65/35) permet d'isoler le produit attendu.
***Point de fusion* : > 258°C (décomposition)**

### EXEMPLE 19 : 3,9-dibromo-12,13-[1,2-(4-O-méthyl-β-D-mannopyranosyl)]-6,7,12,13-tétrahydro(5H)-indolo[2,3-a]pyrrolo[3,4-c]carbazole-5,7-dione

A une solution, refroidie à 0°C, de 0,207 mmol du composé de l'exemple 2 dans 20 ml de tétrahydrofurane est ajoutée goutte à goutte une solution de 2,07 mmol de N-bromosuccinimide dans 30 ml de tétrahydrofurane. Après 7 jours de réaction à température ambiante, le milieu est hydrolysé et extrait à l'acétate d'éthyle. La phase organique est séchée, filtrée puis concentrée sous pression réduite. Une chromatographie sur gel de silice (acétate d'éthyle/cyclohexane : 70/30) permet d'isoler le produit attendu.
**Point de fusion : > 300°C**
**InfraRouge (KBr) : ν**_{**C=O**} **=1710, 1760 cm**^{**-1**}
**ν**_{**NH,OH**} **= 2700-3300 cm**^{**-1**}

### EXEMPLE 20 : 12,13-[1,2-(3-O-bromoacétyl-4-O-méthyl-β-D-mannopyranosyl)]-6,7,12,13-tétrahydro(5H)-indolo[2,3-a]pyrrolo[3,4-c]carbazole-5,7-dione

A une solution de 0,70 mmol du composé de l'exemple 2 dans 12 ml de tétrahydrofurane sont ajoutés 79 mg de tertiobutylate de potassium, puis après 30 minutes à température ambiante, 0,70 mmol de bromure de bromoacétyle. Après 24 heures de réaction, le milieu réactionnel est hydrolysé puis extrait à l'acétate d'éthyle. La phase organique est séchée, filtrée et concentrée sous pression réduite. Une chromatographie sur gel de silice (dichlorométhane/acétate d'éthyle : 70/30) permet d'isoler le produit attendu.

### EXEMPLE 21 : 12,13-[1,2-(6-O-bromoacétyl-4-O-méthyl-β-D-mannopyranosyl)]-6,7,12,13-tétrahydro(5H)-indolo[2,3-a]pyrrolo[3,4-c]carbazole-5,7-dione

On procède comme dans l'exemple 20 en utilisant comme base du carbonate de potassium et en effectuant la réaction au reflux du tétrahydrofurane.

### EXEMPLE 22 : 1,11-dichloro-12,13-[1,2-(6-chloro-6-déoxy-4-O-méthyl-β-D-mannopyranosyl)]-6,7,12,13-térahydro(5H)-indolo[2,3-a] pyrrolo|3,4-c]carbazole-5,7-dione

On procède comme dans l'exemple 10 en utilisant comme substrat le composé de l'exemple 1.

### EXEMPLE 23 : Chlorhydrate de 12,13-[1,2-(6-amine-6-déoxy-4-O-méthyl-β-D-mannopyranosyl)]-6,7,12,13-tétrahydro(5H)-indolo[2,3-a] pyrrolo[3,4-c]carbazole-5,7-dione

A une suspension de 0,126 mmol du composé de l'exemple 12 dans 15 ml de méthanol et 14 ml d'acétate d'éthyle sont ajoutés 7 mg de Pd/C à 10 %. Le mélange est hydrogéné sous une pression d'une atmosphère pendant 40 heures, puis filtré sur célite et lavé successivement avec du méthanol, du tétrahydrofurane et de l'acétate d'éthyle. Le filtrat est concentré sous pression réduite et le résidu obtenu est mis en suspension dans 0,3 ml de méthanol en présence de 0,23 ml d'une solution d'acide chlorhydrique 1N. Après agitation et addition de dichlorométhane, le précipité formé est filtré permettant d'isoler le produit attendu.
**Point de fusion : > 300°C**
**InfraRouge (KBr) : ν**_{**C=O**} **= 1710, 1760 cm**^{**-1**}
**ν**_{**NH,OH**} **= 3270-3600 cm**^{**-1**}

### EXEMPLE 24 : 12,13-[1,2-(6-iodo-6-déoxy-4-O-méthyl-β-D-mannopyranosyl)]-6,7,12,13-tétrahydro(5H)-indolo[2,3-a]pyrrolo[3,4-c]carbazole-5,7-dione

A une solution de 1,55 mmol du composé de l'exemple 10 dans 80 ml d'acétone sont ajoutées 31 mmol d'iodure de sodium. Le milieu est agité à reflux pendant 7 jours, puis le solvant est évaporé. Le résidu est repris à l'acétate d'éthyle, puis lavé. La phase organique est séchée, filtrée et concentrée sous pression réduite. Une chromatographie sur gel de silice (cyclohexane/acétate d'éthyle : 60/40) permet d'isoler le produit attendu.

### EXEMPLE 25 : Chlorhydrate de 12,13-[1,2-(6-diméthylamino-6-déoxy-4-O-méthyl-β-D-mannopyranosyl)]-6,7,12,13-tétrahydro(5H)-indolo[2,3-a] pyrrolo[3,4-c]carbazole-5,7-dione

A une solution de 3,14 mmol de diméthylamine dans 5 ml de tétrahydrofurane est ajoutée 0,154 mmol du composé de l'exemple 24. Après 2 jours d'agitation à température ambiante, le milieu réactionnel est hydrolysé et extrait à l'acétate d'éthyle. La phase organique est séchée, filtrée et concentrée sous pression réduite. Au résidu dilué dans 0,3 ml de méthanol est ajoutée une solution aqueuse d'acide chlorhydrique 1N. Après agitation et addition de dichlorométhane, le précipité formé est filtré permettant d'isoler le produit attendu.

### EXEMPLE 26 : 6-amino-12,13-[1,2-(4-O-méthyl-β-D-mannopyranosyl)]-6,7,12,13-tétrahydro(5H)-indolo[2,3-a]pyrrolo[3,4-c]carbazole-5,7-dione

A 0,20 mmol du composé de l'exemple 2 sont ajoutées 14 mmol d'hydrazine hydraté. Après 1,5 heures d'agitation à 50°C, le mélange réactionnel est jeté sur de la glace, puis extrait à l'acétate d'éthyle. La phase organique est ensuite lavée, séchée, filtrée, puis concentrée sous pression réduite. Une chromatographie sur gel de silice (acétate d'éthyle/cyclohexane) permet d'isoler le produit attendu.

### EXEMPLE 27 : 6-formamido-12,13-[1,2-(4-O-méthyl-β-D-mannopyranosyl)]-6,7,-12,13-tétrahydroindolo[2,3-a]pyrrolo[3,4-c]carbazole-5,7-dione

A une solution de 0,30 mmol du composé de l'exemple 3 dans 10 ml de diméthylformamide sont ajoutés 10 équivalents d'hydrazide formique. Après 1 heure d'agitation à 140°C, le milieu réactionnel est refroidi, puis hydrolysé, entraînant la formation d'un précipité. Celui-ci est filtré et lavé à l'eau puis à l'éther, permettant d'isoler le produit attendu.

### EXEMPLE 28 : 6-(2-hydroxyéthyl)-12,13-[1,2-(4-O-méthyl-β-D-mannopyranosyl)]-6,7,12,13-tétrahydro(5H)-indolo[2,3-a]pyrrolo[3,4-c]carbazole-5,7-dione

Une solution de 0,30 mmol du composé de l'exemple 3 et 1,3 ml d'éthanolamine est agitée 1 heure à température ambiante, puis jetée sur de la glace et extrait à l'acétate d'éthyle. La phase organique est séchée, filtrée puis concentrée sous pression réduite. Une chromatographie sur gel de silice (acétate d'éthyle/cyclohexane) permet d'isoler le produit attendu.

### EXEMPLE 29 : 6-diéthylaminoéthyl-12,13-[1,2-(4-O-méthyl-β-D-mannopyranosyl)]-6,7,12,13-tétrahydro(5H)-indolo[2,3-a]pyrrolo[3,4-c]carbazole-5,7-dione et son chlorhydrate

A une solution de 58,5 mg du composé de l'exemple 3 dissous dans 7 ml de tétrahydrofurane sec, sont ajoutés goutte à goutte 26 µl de N,N-diéthyléthylènediamine. Le mélange est porté à 65°C pendant 4 jours à l'abri de la lumière puis refroidi et repris avec un mélange (solution aqueuse d'acide chlorhydrique 1N / acétate d'éthyle). Après extraction à l'acétate d'éthyle, la phase organique est séchée, filtrée puis concentrée sous pression réduite. Au résidu obtenu, refroidi à 0°C et dissout dans 200 µl de méthanol, est ajoutée goutte à goutte une solution aqueuse d'acide chlorhydrique 1,14N (108 µl). Le mélange est agité, puis du cyclohexane ajouté. Le précipité est filtré sur fritté permettant d'isoler le produit attendu.
**Point de fusion : 250°C**
**InfraRouge (KBr) : ν**_{**C=O**} **= 1700, 1750 cm**^{**-1**}
**ν**_{**NH,OH**} **= 3200-3600 cm**^{**-1**}

### EXEMPLE 30 : 12,13-[1,2-(3,6-di-O-acétyl-4-O-méthyl-β-D-mannopyranosyl)]-6,7, 12,13-tétrahydro(5H)-indolo[2,3-a]pyrrolo[3,4-c]carbazole-5,7-dione

A une solution de 351 mg du composé de l'exemple 2 dans 7 ml de pyridine, est ajouté goutte à goutte 0, 68 ml d'anhydride acétique .Le mélange réactionnel est agité 19 heures à température ambiante. Après hydrolyse, le produit organique est extrait avec de l'acétate d'éthyle puis la phase organique est lavée, séchée. Après évaporation du solvant, le résidu est purifié par chromatographie sur gel de silice (cyclohexane/acétate d'éthyle : 3/2), permettant d'isoler le produit attendu.
**Point de fusion : 106°C**
**InfraRouge (KBr) : ν**_{**C=O**} **= 1720, 1750 cm**^{**-1**}
**ν**_{**NH,OH**} **= 3100-3600 cm**^{**-1**}

### EXEMPLE 31 : 12,13-[1,2-(3-O-acétyl-4-O-méthyl-β-D-mannopyranosyl)]-6,7,12,13-tétrahydro(5H)-indolo[2,3-a]pyrrolo[3,4-c]carbazole-5,7-dione

A une solution, refroidie à 0°C, de 337 mg du composé de l'exemple 30 dissous dans 30 ml d'acétonitrile et 3 ml d'eau, est ajouté goutte à goutte 0,92 ml de trifluorure de bore étherate. Après 24 heures à température ambiante, 3 ml d'eau et 0,92 ml de trifluorure de bore étherate sont rajoutés. Après à nouveau 24 heures à température ambiante, la réaction est hydrolysée avec une solution aqueuse saturée de bicarbonate de sodium. Le produit organique est extrait avec de l'acétate d'éthyle puis les phases organiques rassemblées, lavées et séchées. Après évaporation du solvant, une chromatographie sur gel de silice (cyclohexane/acétate d'éthyle 1/1 permet d'isoler le produit attendu.
**Point de fusion : 294°C**
**InfraRouge (KBr) : ν**_{**C=O**} **= 1720, 1750 cm**^{**-1**}
**ν**_{**NH,OH**} **= 3100-3600 cm**^{**-1**}

### EXEMPLE 32 : 12,13-[1,2-(3-O-acétyl-6-déoxy-6-chloro-4-O-méthyl-β-D-manno pyranosyl)]-6,7,12,13-tétrahydro(5H)-indolo[2,3-a]pyrrolo[3,4-c] carbazole-5,7-dione

A une solution de 77 mg du composé de l'exemple 31 dans 1,7 ml de pyridine, sont ajoutés successivement 154 mg de triphénylphosphine puis goutte à goutte le tétrachlorure de carbone (43 µl). Le mélange réactionnel est agité 65 heures à 40°C, refroidi puis versé dans de l'eau (30 ml). Le produit organique est extrait avec de l'acétate d'éthyle, lavé puis séché sur sulfate de magnésium. Après évaporation du solvant, le résidu est purifié par chromatographie sur gel de silice (cyclohexane/acétate d'éthyle : 55/45) permettant d'isoler le produit attendu
**Point de fusion : > 300°C**
**InfraRouge (KBr) : ν**_{**C=O**} **= 1700, 1730, 1770 cm**^{**-1**}
**ν**_{**NH**} **= 3100-3600 cm**^{**-1**}

### EXEMPLE 33 : 3,9-dinitro-12,13-[1,2-(3-O-acétyl-6-déoxy-6-chloro-4-O-méthyl-β-D-mannopyranosyl)]-6,7,12,13-tétrahydro(5H)-indolo[2,3-a]pyrrolo [3,4-c]carbazole-5,7-dione

A une solution refroidie à 0°C de 50,6 mg du composé de l'exemple 32 dissous dans 5 ml de térahydrofuranne sec est ajouté goutte à goutte 3,5 ml d'acide nitrique fumant. Après 2 heures, le mélange est remonté à température ambiante et laissé sous agitation pendant 21 heures. Après hydrolyse, le produit organique est extrait avec de l'acétate d'éthyle. Les phases organiques sont rassemblées, lavées, séchées sur sulfate de magnésium, et le solvant est évaporé. Une chromatographie sur gel de silice (toluène/tétrahydrofuranne 7/3) conduit au produit dinitré attendu.

### EXEMPLE 34 : 3,9-dinitro-12,13-[1,2-(6-déoxy-6-chloro-4-O-méthyl-β-D-manno pyranosyl)]-6,7,12,13-tétrahydro(5H)-indolo[2,3-a]pyrrolo[3,4-c] carbazole-5,7-dione

A une solution, refroidie à 0°C, de 22,3 mg du composé de l'exemple 33 dissous dans 3 ml d'acétonitrile, 0,3 ml d'eau et 2 ml de tétrahydrofuranne, est ajouté goutte à goutte le trifluorure de bore étherate (48 µl). Après 48 heures à 40°C, 0,3 ml d'eau et 1 ml de trifluorure de bore étherate sont rajoutés. Après à nouveau 24 heures à 40°C, les mêmes proportions d'eau et de trifluorure de bore étherate sont ajoutés. Après 24 heures à température ambiante, la réaction est hydrolysée avec une solution aqueuse saturée de bicarbonate de sodium. Le produit organique est extrait avec de l'acétate d'éthyle puis les phases organiques rassemblées, lavées et séchées sur sulfate de magnésium. Après évaporation du solvant, une chromatographie sur gel de silice (toluène/tétrahydrofuranne :1/1), puis (cyclohexane/acétone : 2/3) permet d'isoler le produit attendu.

### EXEMPLE 35 : 3,9-dinitro-12,13-[1,2-(6-azido-6-déoxy-4-O-méthyl-β-D-manno pyranosyl)]-6,7,12,13-tétrahydro(5H)-indolo[2,3-a]pyrrolo[3,4-c] carbazole-5,7-dione

On procède comme dans l'exemple 10 en utilisant comme produit de départ le composé de l'exemple 34.
**Point de fusion : > 300°C**
**InfraRouge (KBr) : ν**_{**C=O**} **= 1720, 1760 cm**^{**-1**}
**ν**_{**NH**} **= 3200-3600 cm**^{**-1**}
**ν**_{**N3**} **= 2100 cm**^{**-1**}

### EXEMPLE 36 : 3,9-dicarbométhoxy-12,13-[1,2-(4-O-méthyl-β-D-mannopyranosyl)] -6,7,12,13-tétrahydro(5H)-indolo[2,3-a]pyrrolo[3,4-c]carbazole -5,7-dione

Un mélange de 290 mg composé de l'exemple 2 et de 6 ml de pyridine est refroidi à 0°C puis 0,6 ml d'anhydride acétique est additionné. Le mélange réactionnel est agité à température ambiante pendant 18 heures. De l'eau est ajoutée et le mélange est agité pendant 40 minutes, puis extrait avec de l'acétate d'éthyle. La phase organique est lavée puis séchée sur MgSO₄. Le solvant est évaporé et le résidu dissous dans 9 ml de dichlorométhane. Du dichlorométhylméthyléther (1,08ml) est ajouté et le mélange est refroidi à 0°C avant l'addition d'une solution 1M de TiCl₄ dans 11,96 ml de dichlorométhane. Le mélange est agité à température ambiante pendant 24 heures puis hydrolysé avec de l'eau. Le mélange réactionnel est agité pendant une heure puis il est extrait avec de l'acétate d'éthyle. La phase organique est séchée sur MgSO₄ et le solvant est évaporé. Le résidu est alors dissout dans 6 ml de méthanol avant addition d' une solution à 50 % dans l'eau de peroxyde d'hydrogène (0,4 ml) puis d'acide sulfurique à 95 % (1,6 ml). Le mélange est agité 72 heures à température ambiante. Après 30 minutes d'hydrolyse le mélange réactionnel est extrait avec de l'acétate d'éthyle. Les phases organiques sont rassemblées, séchées sur sulfate de magnésium, filtrées et le solvant évaporé. Le résidu ainsi obtenu est ensuite dissout dans 22 ml de méthanol avant addition goutte à goutte d'une solution aqueuse à 28 % d'hydroxyde d'ammonium (10 ml). Le mélange est agité 24 heures à température ambiante. Après évaporation des solvants, le résidu est repris avec un mélange eau / acétate d'éthyle. Le mélange réactionnel est extrait avec de l'acétate d'éthyle. Les phases organiques sont rassemblées, lavées avec une solution aqueuse de chlorure de sodium, séchées sur sulfate de magnésium, filtrées et le solvant évaporé. Le résidu purifié par chromatographie sur gel de silice (cyclohexane/acétone : 1/1) conduit au produit attendu.

### EXEMPLE 37 : N³,N⁹-bis(3-aminopropyl)-5,7-dioxo-12,13-[1,2-(4-O-méthyl-β-D-mannopyranosyl)]-6,7,12,13-tétrahydro(5H)-indolo[2,3-a]pyrrolo [3,4-c]carbazole-3,9-dicarboxamide

0,2 ml de 1,3-diaminopropane est ajouté à une solution de 1,26 ml de Al(CH₃)₃ 2N dans l'hexane à -20°C. Le mélange est agité pendant 20 minutes à -20°C puis ramené lentement à température ambiante. 1,36 g du composé de l'exemple 7 dissous dans 15 ml de dichlorométhane est ajouté et la solution est chauffée à reflux pendant 24 heures. Le mélange réactionnel est hydrolysé à l'aide d'une solution de 4 ml d'acide chlorhydrique 0,7 M. Après agitation pendant 30 minutes, la phase aqueuse est séparée et extraite avec de l'acétate d'éthyle. La phase organique est lavée, puis séchée. Après évaporation du solvant, le résidu obtenu est purifié par chromatographie sur colonne de silice pour conduire au produit attendu.

### EXEMPLE 38 : 3,9-dichloro-12,13-[1,2-(4-O-méthyl-β-D-mannopyranosyl)]-6,7,12,13 -tétrahydro(5H)-indolo[2,3-a]pyrrolo[3,4-c]carbazole-5,7-dione

A une solution, refroidie à 0°C, de 60 mg du composé de l'exemple 2 dans 3 ml de tétrahydrofurane est ajoutée goutte à goutte une solution de 166 mg de N-chlorosuccinimide dissous dans 6 ml de tétrahydrofurane. Le mélange est agité 4 jours à température ambiante. Après 10 minutes d'hydrolyse par 50 ml d'eau, le dérivé dichloré est extrait avec de l'acétate d'éthyle La phase organique est séchée, filtrée et le solvant évaporé. Le résidu purifié par chromatographie sur gel de silice (cyclohexane/acétate d'éthyle : 3/7) conduit au produit attendu.

### EXEMPLE 39 : 3,9-diamino-12,13-[1,2-(4-O-méthyl-β-D-mannopyranosyl)]-6,7,12,13 -tétrahydro-5-oxo(5H)-indolo[2,3-a]pyrrolo[3,4-c]carbazole et 3,9-diamino-12,13-[1,2-(4-O-méthyl-β-D-mannopyranosyl)]-6,7,12,13-tétrahydro-7-oxo(5H)-indolo[2,3-a]pyrrolo[3,4-c]carbazole

1 g d'amalgame de zinc est ajouté à une solution de 106 mg du composé de l'exemple 15 dans 14 ml d'éthanol en présence de 2,3 ml d'acide chlorhydrique 6 N. Le mélange est porté à reflux pendant 4 heures puis filtré. Le résidu solide est lavé avec de l'acétate d'éthyle. Le filtrat est évaporé, le résidu est repris avec de l'acétate d'éthyle, lavé avec une solution aqueuse saturée de NaHCO₃, puis avec de l'eau. La phase organique est séchée sur MgSO₄. Le solvant est évaporé et le résidu est purifié par chromatographie sur colonne de silice (acétate d'éthyle/méthanol : 9/1) permettant d'isoler un mélange des régioisomères attendus.

### EXEMPLE 40 : 12,13-[1,2-(3-O-lysine-4-O-méthyl-β-D-mannopyranosyl)]-6,7,12,13-tétrahydro(5H)-indolo[2,3-a]pyrrolo[3,4-c]carbazole-5,7-dione et son chlorhydrate

A une solution de 10 ml de lysine-diterbutoxycarbonyl dans le diméthylformamide, refroidie à 0°C sont ajoutés 68 mg d'hydroxybenzotriazole et 114 mg de dicyclohexylcarbodiimide. Le mélange est agité à température ambiante pendant 30 minutes avant d'être ajouté à une suspension de 242 mg du composé de l'exemple 2 dans 5 ml de tétrahydrofurane, cette solution étant maintenue au préalable sous agitation à température ambiante en présence de 69 mg de carbonate de potassium. Le mélange réactionnel ainsi obtenu est chauffé à 50°C pendant 24 heure avant d'être hydrolysé avec une solution saturée de NaCl puis extrait à l'acétate d'éthyle. Les phases organiques rassemblées sont séchées puis filtrées et concentrées sous pression réduite. Une chromatographie sur gel de silice du résidu (cyclohexane/acétate d'éthyle : 3/7) permet d'isoler un composé qui est alors mis en solution dans un mélange 3M d'HCl dans 2,5 ml d'acétate d'éthyle. Après 5 heures d'agitation à température ambiante, le mélange réactionnel est évaporé pour donner des cristaux qui sont lavés avec de l'acétate d'éthyle pour conduire au produit attendu sous forme de chlorhydrate.

### ETUDE PHARMACOLOGIQUE DES COMPOSES DE L'INVENTION

### EXEMPLE 41 : Activité in vitro

### • Leucémie murine L1210

La leucémie murine L1210 a été utilisée in vitro. Les cellules sont cultivées dans du milieu de culture RPMI 1640 complet contenant 10 % de sérum de veau foetal, 2 mM de glutamine, 50 U/ml de pénicilline, 50 µg/ml de streptomycine et 10 mM d'Hepes, pH : 7,4. Les cellules sont réparties dans des microplaques et exposées aux composés cytotoxiques pendant 4 temps de doublement, soit 48 heures. Le nombre de cellules viables est ensuite quantifié par un essai colorimétrique, le Microculture Tetrazolium Assay (J. Carmichael et al., Cancer Res.; 47, 936-942, (1987)). Les résultats sont exprimés en IC₅₀, concentration en cytotoxique qui inhibe à 50 % la prolifération des cellules traitées. Tous les produits de l'invention montrent une bonne cytotoxicité sur cette lignée cellulaire. A titre illustratif, le composé de l'exemple 13, lors de ce test, présente un IC₅₀ de 0,13 µM et celui de l'exemple 19 un IC₅₀ de 0,14 µM.

### • Lignées cellulaires humaines

Les composés de l'invention ont également été testés sur des lignées cellulaires humaines selon le même protocole expérimental que celui décrit sur la leucémie murine L1210 mais avec des temps d'incubation de 4 jours au lieux de 2 jours. A titre indicatif, les composés des exemples 14, 16, 19 et 29 présentent tous des IC₅₀ inférieurs à 1 µM sur les lignées cellulaire suivantes : carcinome ovarien IGROV-1, neuroblastome SK-N-MC, carcinome du colon HT-29, carcinome pulmonaire non à petites cellules A549, carcinome epidermoïde A431 et carcinome pulmonaire à petites cellules H 69. Ces différents résultats démontrent clairement le fort potentiel anti-tumoral des composés de l'invention.

### EXEMPLE 42 : Action sur le cycle cellulaire

Les cellules L1210 sont incubées pendant 21 heures à 37°C en présence de différentes concentrations en produit testés. Les cellules sont ensuite fixées par de l'éthanol à 70 % (v/v), lavées deux fois dans du PBS et incubées 30 minutes à 20°C dans du PBS contenant 100 µg/ml de RNAse et 50 µg/ml d'iodure de propidium. Les résultats sont exprimés en pourcentage des cellules accumulées en phase G2+M après 21 heures par rapport au témoin (témoin : 20 %). Les composés de l'invention sont particulièrement intéressants. Ainsi, ils induisent une accumulation d'au moins 70 % des cellules en phase G2 + M après 21 heures à une concentration inférieure à 1 µM.

### EXEMPLE 43 : Composition pharmaceutique : soluté injectable

Composé de l'exemple 13 10 mg

Eau distillée pour préparations injectables 25 ml

## Revendications

1. Composés de formule (I): 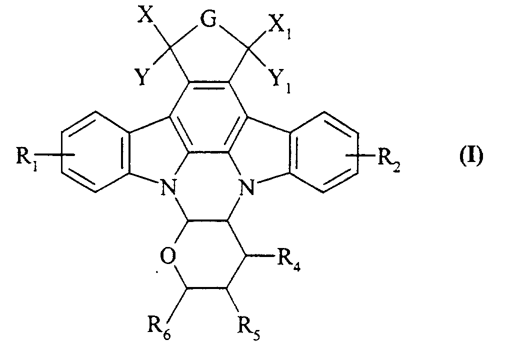 dans laquelle :
• **R**_{**1**}**, R**_{**2**}, identiques ou différents, indépendamment l'un de l'autre, représentent un groupement de formule U-V dans laquelle :
* U représente une liaison simple ou une chaîne alkylène (C₁-C₆) linéaire ou ramifiée, éventuellement substituée par un ou plusieurs groupements, identiques ou différents, choisis parmi halogène et hydroxy, et/ou contenant éventuellement une ou plusieurs insaturations,
* V représente un groupement choisi parmi atome d'hydrogène, d'halogène, groupement cyano, nitro, azido, alkyle (C₁-C₆) linéaire ou ramifié, aryle, arylalkyle (C₁-C₆) linéaire
ou ramifié, hydroxy, alkoxy (C₁-C₆) linéaire ou ramifié, aryloxy, arylalkoxy (C₁-C₆) linéaire ou ramifié, formyle, carboxy, aminocarbonyle, NRaRb, -C(O)-T₁, -C(O)-NRa-T₁, -NRa-C(O)-T₁, -O-C(O)-T₁, -C(O)-O-T₁, -O-T₂-NRaRb, -O-T₂-ORa, -O-T₂-CO₂Ra, -NRa-T₂-NRaRb, -NRa-T₂-ORa, -NRa-T₂-CO₂Ra, et -S(O)ₙ-Ra,
dans lesquels :
→ Ra et Rb, identiques ou différents, représentent chacun un groupement choisi parmi atome d'hydrogène, groupement alkyle (C₁-C₆) linéaire ou ramifié, aryle, et arylalkyle (C₁-C₆) linéaire ou ramifié,
ou
Ra+Rb forment ensemble, avec l'atome d'azote qui les portent, un hétérocycle de 5 à 7 chaînons, monocyclique, contenant éventuellement au sein du système cyclique un second hétéroatome choisi parmi oxygène et azote, et étant éventuellement substitué par un groupement choisi parmi alkyle (C₁-C₆) linéaire ou ramifié, hydroxy, alkoxy (C₁-C₆) linéaire ou ramifié, amino, alkylamino (C₁-C₆) linéaire ou ramifié, et dialkylamino (C₁-C₆) linéaire ou ramifié,
→ T₁ représente un groupement choisi parmi alkyle (C₁-C₆) linéaire ou ramifié, aryle, arylalkyle (C₁-C₆) linéaire ou ramifié, et une chaîne alkylène (C₁-C₆) linéaire ou ramifié substituée par un groupement choisi parmi -ORa, -NRaRb, -CO₂Ra, -C(O)Ra et -C(O)NRaRb dans lesquels Ra et Rb sont tels que définis précédemment,
→ T₂ représente une chaîne alkylène (C₁-C₆) linéaire ou ramifiée,
→ n représente un entier compris entre 0 et 2 inclus,
• **G** représente un atome d'oxygène ou un groupement NR₃ dans lequel R₃ représente un groupement choisi parmi atome d'hydrogène, groupement alkyle (C₁-C₆) linéaire ou ramifié, aryle, arylalkyle (C₁-C₆) linéaire ou ramifié, cycloalkyle, cycloalkylalkyle (C₁-C₆) linéaire ou ramifié, -ORa, -NRaRb, -O-T₂-NRaRb, -NRa-T₂-NRaRb, hydroxyalkylamino (C₁-C₆) linéaire ou ramifié, di(hydroxyalkyl)amino (C₁-C₆) linéaire ou ramifié, -C(O)-Ra, -NH-C(O)-Ra, et une chaîne alkylène (C₁-C₆) linéaire ou ramifié, substituée par un ou plusieurs groupements, identiques ou différents, choisis parmi atomes d'halogène, groupements cyano, nitro, -ORa, -NRaRb, -CO₂Ra, -C(O)Ra, hydroxyalkylamino (C₁-C₆) linéaire ou ramifié, di(hydroxyalkyl)amino (C₁-C₆) linéaire ou ramifié, et -C(O)-NHRa, les groupements Ra, Rb et T₂ ayant les mêmes significations que précédemment,
• **X** représente un groupement choisi parmi atome d'hydrogène, groupement hydroxy, alkoxy (C₁-C₆) linéaire ou ramifié, mercapto, et alkylthio (C₁-C₆) linéaire ou ramifié,
• **Y** représente un atome d'hydrogène,
ou
**X** et **Y** forment ensemble, avec l'atome de carbone qui les porte, un groupement carbonyle,
• **X**_{**1**} représente un groupement choisis parmi atome d'hydrogène, groupement hydroxy, alkoxy (C₁-C₆) linéaire ou ramifié, mercapto, et alkylthio (C₁-C₆) linéaire ou ramifié,
• **Y**_{**1**} représente un atome d'hydrogène,
ou
**X**_{**1**} et **Y**_{**1**} forment ensemble, avec l'atome de carbone qui les porte, un groupement carbonyle,
• **R**_{**4**}**, R**_{**5**}**,** identiques ou différents, indépendamment l'un de l'autre, représentent chacun un groupement choisi parmi atome d'hydrogène, atome d'halogène, groupement hydroxy, alkoxy (C₁-C₆) linéaire ou ramifié, aryloxy, arylalkoxy (C₁-C₆) linéaire ou ramifié, alkyle (C₁-C₆) linéaire ou ramifié, arylalkyle (C₁-C₆) linéaire ou ramifié, aryle, amino (lui-même étant éventuellement substitué par un ou deux groupements, identiques ou différents, choisis parmi alkyle (C₁-C₆) linéaire ou ramifié, aryle, et arylalkyle (C₁-C₆) linéaire ou ramifié), azido, -N=NRa (dans lequel Ra est tel que défini précédemment), et -O-C(O)-Rc dans lequel Rc représente un groupement alkyle (C₁-C₆) linéaire ou ramifié (éventuellement substitué par un ou plusieurs groupements choisis parmi halogène, hydroxy, amino, alkylamino (C₁-C₆) linéaire ou ramifié, et dialkylamino (C₁-C₆) linéaire ou ramifié), aryle, arylalkyle (C₁-C₆) linéaire ou ramifié, cycloalkyle, ou hétérocycloalkyle,
• **R**_{**6**} représente un groupement de formule -U₁-R₄ dans laquelle U₁ représente une liaison simple ou un groupement méthylène, et R₄ est tel que défini précédemment,
• ou **R**_{**4**}**, R**_{**5**}**, R**_{**6**}**,** pris deux à deux en positions adjacentes ou non adjacentes, forment avec les atomes de carbone qui les portent, un système cyclique de 3 à 6 chaînons, contenant un ou deux atomes d'oxygène, le groupement restant R₄, R₅ ou R₆ n'appartenant pas au dit système cyclique prenant alors l'une quelconque des définitions de R₄, R₅, ou R₆ précisées précédemment,
leurs isomères, ainsi que leurs sels d'addition à un acide ou à une base pharmaceutiquement acceptable,
étant entendu que les composés de formule (I) sont différents des composés suivants :
- le 1,11-dichloro-12,13-[1,2-(4-O-méthyl-β-D-mannopyranosyl)]-6,7,12,13-tétrahydro (5*H*)-indolo[2,3-a]pyrrolo[3,4-c]carbazole-5,7-dione,
- le 12,13-[1,2-(4-O-méthyl-β-D-mannopyranosyl)-6,7,12,13-tétrahydro(5*H*)-indolo[2,3-a] pyrrolo[3,4-c]carbazole-5,7-dione,
- le 12,13-[1,2-(4-O-méthyl-β-D-mannopyranosyl)-6,7,12,13-tétrahydro(5*H*)-indolo[2,3-a] pyrrolo[3,4-c]carbazole-5-one,
- et le 12,13-[1,2-(4-O-méthyl-β-D-mannopyranosyl)-5,6,12,13-tétrahydro(7*H*)-indolo [2,3-a]pyrrolo[3,4-c]-carbazole-7-one,
étant entendu également que par :
- cycloalkyle, on comprend un groupement monocyclique ou bicyclique, saturé ou insaturé mais sans caractère aromatique, comportant de 3 à 10 atomes de carbone, étant éventuellement substitué par un ou plusieurs groupements, identiques ou différents, choisis parmi halogène, alkyle (C₁-C₆) linéaire ou ramifié, trihalogénoalkyle (C₁-C₆) linéaire ou ramifié, hydroxy, alkoxy (C₁-C₆) linéaire ou ramifié, et amino éventuellement substitué par un ou deux groupements alkyle (C₁-C₆) linéaire ou ramifié,
- hétérocycloalkyle, on comprend un groupement cycloalkyle tel que défini précédemment comportant au sein du système cyclique un ou deux hétéroatomes, identiques ou différents, choisis parmi oxygène, azote et soufre,
- aryle, on comprend un groupement phényle, naphtyle, dihydronaphtyle, tétrahydronaphtyle, indényle ou indanyle, chacun de ces groupements étant éventuellement substitué par un ou plusieurs groupements, identiques ou différents, choisis parmi halogène, alkyle (C₁-C₆) linéaire ou ramifié, trihalogénoalkyle (C₁-C₆) linéaire ou ramifié, hydroxy, alkoxy (C₁-C₆) linéaire ou ramifié, et amino éventuellement substitué par un ou deux groupements alkyle (C₁-C₆) linéaire ou ramifié.

2. Composés de formule (I) selon la revendication 1 **caractérisés en ce que** G représente un groupement NR₃ dans lequel R₃ est tel que défini dans la formule (I), leurs isomères, ainsi que leurs sels d'addition à un acide ou à une base pharmaceutiquement acceptable.

3. Composés de formule (I) selon la revendication 1 **caractérisés en ce que** X et Y forment ensemble, avec l'atome de carbone qui les porte, un groupement carbonyle, et X₁ et Y₁ forment ensemble avec l'atome de carbone qui les porte, un groupement carbonyle, leurs isomères, ainsi que leurs sels d'addition à un acide ou à une base pharmaceutiquement acceptable.

4. Composés de formule (I) selon la revendication 1 **caractérisés en ce qu'**ils représentent des composés de formule (Ibis) : 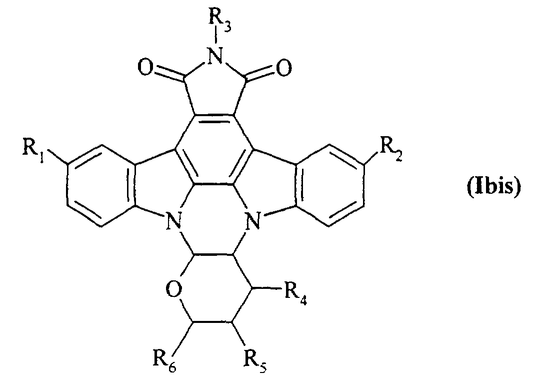 dans laquelle R₁, R₂, R₃, R₄, R₅ et R₆ sont tels que définis dans la formule (I), leurs isomères, ainsi que leurs sels d'addition à un acide ou à une base pharmaceutiquement acceptable.

5. Composés de formule (I) selon la revendication 1 ou 4 **caractérisés en ce que** R₁ et R₂, identiques ou différents, représentent un groupement de formule U-V dans laquelle U représente une liaison simple et V représente un groupement choisi parmi atome d'halogène, atome d'hydrogène, groupement nitro, formyle, hydroxy, et une chaîne alkylène (C₁-C₆) linéaire ou ramifiée substituée par un groupement hydroxy, leurs isomères, ainsi que leurs sels d'addition à un acide ou à une base pharmaceutiquement acceptable.

6. Composés de formule (I) selon la revendication 1 ou 4 **caractérisés en ce que** R₁ et R₂ sont identiques, leurs isomères ainsi que leurs sels d'addition à un acide ou à une base pharmaceutiquement acceptable.

7. Composés de formule (I) selon la revendication 1 ou 4 **caractérisés en ce que** R₃ représente l'atome d'hydrogène, un groupement hydroxy ou un groupement chaîne alkylène (C₁-C₆) linéaire ou ramifié substituée par un groupement choisi parmi NRaRb et ORa dans lesquels Ra et Rb sont tels que définis dans la formule (I), leurs isomères, ainsi que leurs sels d'addition à un acide ou à une base pharmaceutiquement acceptable.

8. Composés de formule (I) selon la revendication 1 ou 4 **caractérisés en ce qu'**ils représentent des composés de formule (Iter) : 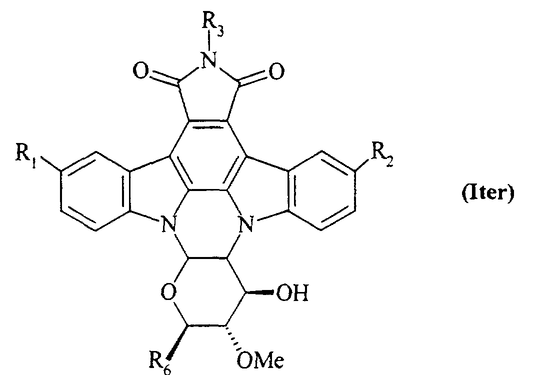 dans laquelle R₁, R₂, R₃ et R₆ sont tels que définis dans la formule (I), leurs isomères, ainsi que leurs sels d'addition à un acide ou à une base pharmaceutiquement acceptable.

9. Composés de formule (I) qui sont le :
- 3,9-diformyl-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione,
- 3,9-dinitro-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione,
- 3-nitro-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione,
-9-nitro-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione,
-6-hydroxy-12,13-[1,2-(4-O-méthyl-β-D-mannopyranosyl)]-6,7,12,13-tétrahydro(5*H*)-indolo[2,3-*a*]pyrrolo[3,4-*c*]carbazole-5,7-dione,
- 3,9-di-(hydroxyméthyl)-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione,
- 3,9-dinitro-12,13-[1,2-(3,6-anhydro-4-*O*-méthyl-β-D-mannopyranosyl)]-6,7,12,13-tétrahydro(5*H*)-indolo[2,3-*a*]pyrrolo[3,4-*c*]carbazole-5,7-dione,
- 3,9-dihydroxy-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione,
- 3,9-dibromo-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione,
- et le 6-diéthylaminoéthyl-12,13-[1,2-(4-*O*-méthyl-β-D-mannopyranosyl)]-6,7,12,13-tétrahydro(5*H*)-indolo[2,3-*a*]pyrrolo[3,4-*c*]carbazole-5,7-dione et son chlorhydrate. leurs isomères, ainsi que leurs sels d'addition à un acide ou à une base pharmaceutiquement acceptable.

10. Procédé de préparation des composés de formule (I), **caractérisé en ce qu'**on utilise, comme produit de départ, un composé de formule (II) : 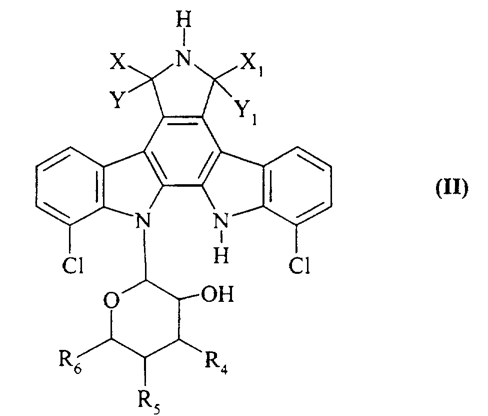 dans laquelle X, Y, X₁, Y₁, R₄, R₅ et R₆ sont tels que définis dans la formule (I), que l'on traite en milieu basique en présence d'acide *para*-toluènesulfonique, pour conduire au composé de formule (III): 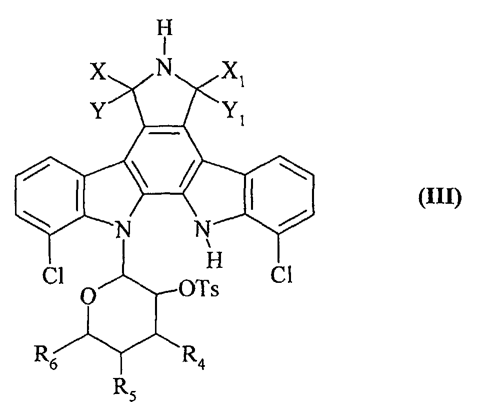 dans laquelle X, Y, X₁, Y₁, R₄, R₅ et R₆ sont tels que définis précédemment, composé de formule (III) qui est mis à réagir avec de l'azoture de sodium, pour conduire principalement au composé de formule (I/a), cas particulier des composés de formule (I) : 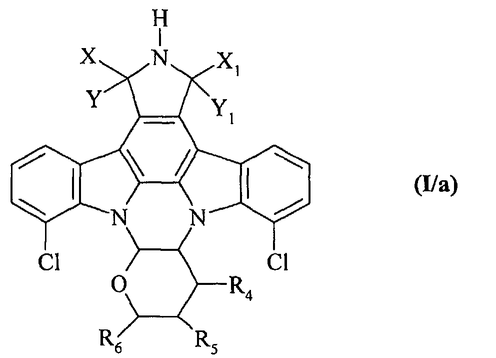 dans laquelle X, Y, X₁, Y₁, R₄, R₅ et R₆ sont tels que définis précédemment, composé de formule (I/a) qui est éventuellement soumis à des conditions d'hydrogénolyse, pour conduire au composé de formule (I/b), cas particulier des composés de formule (I): 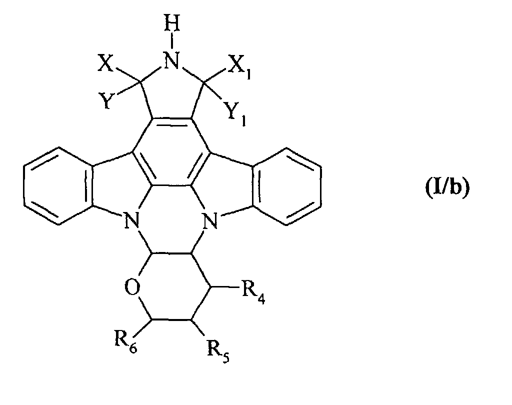 dans laquelle X, Y, X₁, Y₁, R₄, R₅ et R₆ sont tels que définis précédemment, composé de formule (I/b) qui est éventuellement traité par de la soude aqueuse puis placé en présence d'acide chlorhydrique, pour conduire au composé de formule (I/c), cas particulier des composés de formule (I) : 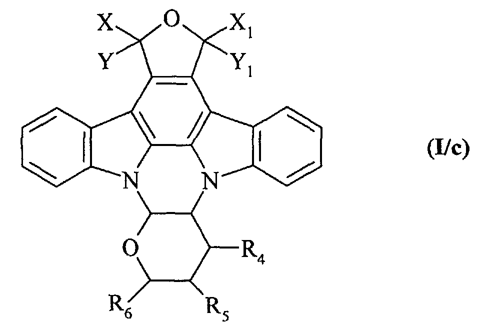 dans laquelle X, Y, X₁, Y₁, R₄, R₅ et R₆ sont tels que défmis précédemment, composé de formule (I/c) qui est éventuellement soumis à l'action d'un composé de formule (IV) :
R₃ₐ - NH₂**(IV)**
dans laquelle R₃ₐ a la même définition que R₃ à l'exception de la définition atome d'hydrogène, pour conduire au composé de formule (I/d), cas particulier des composés de formule (I): 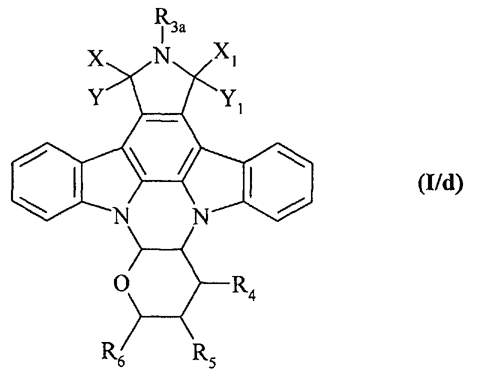 dans laquelle X, Y, X₁, Y₁, R₃ₐ, R₄, R₅ et R₆ sont tels que définis précédemment, l'ensemble des composés de formule (I/b) à (I/d) formant les composés de formule (I/e): 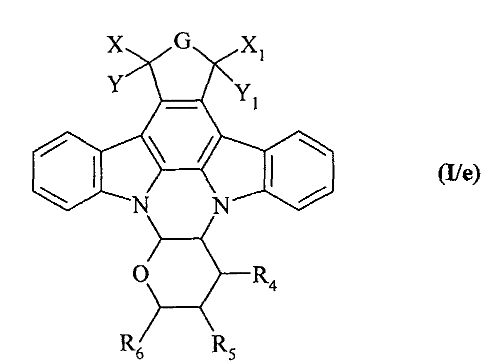 dans laquelle G, X, Y, X₁, Y₁, R₄, R₅ et R₆ sont tels que définis dans la formule (I), composé de formule (I/e) qui est éventuellement soumis à une réaction d'addition électrophile aromatique ou d'addition nucléophile aromatique, selon des conditions classiques de la synthèse organique et bien connues de l'homme de l'art, pour conduire au composé de formule (I/f), cas particulier des composés de formule (I): 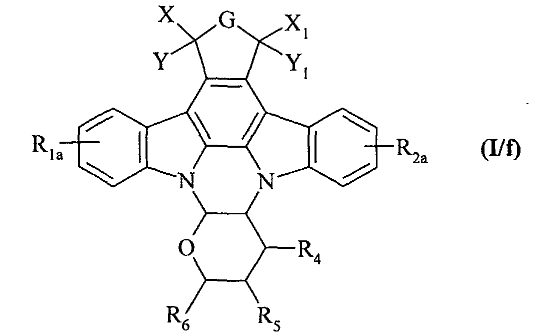 dans laquelle G, X, Y, X₁, Y₁, R₄, R₅ et R₆ sont tels que définis précédemment, et R₁ₐ et R₂ₐ ont les mêmes définitions réciproques que R₁ et R₂ à l'exception que R₁ₐ et R₂ₐ ne peuvent pas représenter simultanément un atome d'hydrogène,
les composés de formule (I/a) à (I/f) formant l'ensemble des composés de formule (I), que l'on purifie, le cas échéant, selon des techniques classiques de purification, qui peuvent, si on le désire, être séparés en leurs différents isomères, selon une technique classique de séparation, dont on module les substituants R₄, R₅ et R₆ selon les méthodes classiques de la synthèse organique utilisées dans le domaine de la chimie des sucres, et que l'on transforme, si on le souhaite, en leurs sels d'addition à un acide ou à une base pharmaceutiquement acceptable.

11. Compositions pharmaceutiques contenant comme principe actif au moins un composé de formule (I) selon l'une quelconque des revendications 1 à 9, seul ou en combinaison avec un ou plusieurs excipients ou véhicules inertes, non toxiques, pharmaceutiquement acceptables.

12. Compositions pharmaceutiques selon la revendication 10, utile en tant qu'agents antitumoraux dans le traitement des cancers.

## Claims

1. Compounds of formula (I): 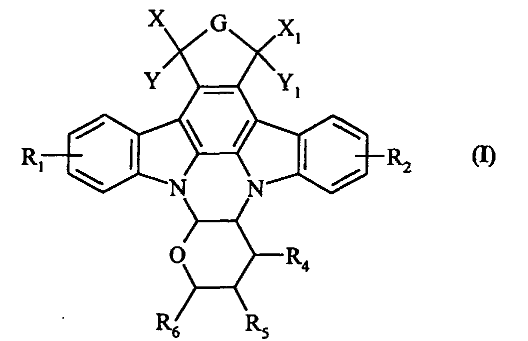 wherein:
• **R**_{**1**} and **R**_{**2**}**,** which may be identical or different, each independently of the other represents a group of formula U-V wherein :
* U represents a single bond or a linear or branched (C₁-C₆)alkylene chain, optionally substituted by one or more identical or different groups selected from halogen and hydroxy, and/or optionally containing one or more unsaturations,
* V represents a group selected from a hydrogen atom, a halogen atom, a cyano group, a nitro group, an azido group, a linear or branched (C₁-C₆)alkyl group, an aryl group, an aryl-(C₁-C₆)alkyl group in which the alkyl moiety is linear or branched, a hydroxy group, a linear or branched (C₁-C₆)alkoxy group, an aryloxy group, an aryl-(C₁-C₆)-alkoxy group in which the alkoxy moiety is linear or branched, a formyl group, a carboxy group, an aminocarbonyl group, an NRaRb, -C(O)-T₁, -C(O)-NRa-T₁, -NRa-C(O)-T₁, -O-C(O)-T₁, -C(O)-O-T₁, -O-T₂-NRaRb, -O-T₂-ORa, -O-T₂-CO₂Ra, -NRa-T₂-NRaRb, -NRa-T₂-ORa, -NRa-T₂-CO₂Ra and an -S(O)ₙ-Ra group,
wherein:
→ Ra and Rb, which may be identical or different, each represents a group selected from a hydrogen atom, a linear or branched (C₁-C₆)alkyl group, an aryl group and an aryl-(C₁-C₆)alkyl group in which the alkyl moiety is linear or branched,
or
Ra+Rb form together with the nitrogen atom carrying them a monocyclic heterocycle having from 5 to 7 ring members, optionally containing within the ring system a second hetero atom selected from oxygen and nitrogen, and optionally being substituted by a group selected from linear or branched (C₁-C₆)alkyl, hydroxy, linear or branched (C₁-C₆)alkoxy, amino, linear or branched (C₁-C₆)alkyl-amino and di-(C₁-C₆)alkylamino in which each alkyl moiety is linear or branched,
→ T₁ represents a group selected from linear or branched (C₁-C₆)alkyl, aryl, aryl-(C₁-C₆)alkyl in which the alkyl moiety is linear or branched, and a linear or branched (C₁-C₆)alkylene chain substituted by a group selected from -ORa, -NRaRb, -CO₂Ra, -C(O)Ra and -C(O)NRaRb wherein Ra and Rb are as defined hereinbefore,
→ T₂ represents a linear or branched (C₁-C₆)alkylene chain,
→ n represents an integer from 0 to 2 inclusive,
• **G** represents an oxygen atom or an NR₃ group wherein R₃ represents a group selected from a hydrogen atom, a linear or branched (C₁-C₆)alkyl group, an aryl group, an aryl-(C₁-C₆)alkyl group in which the alkyl moiety is linear or branched, a cycloalkyl group, a cycloalkyl-(C₁-C₆)alkyl group in which the alkyl moiety is linear or branched, an -ORa group, an -NRaRb group, an -O-T₂-NRaRb group, an -NRa-T₂-NRaRb group, a (C₁-C₆)hydroxyalkyl-amino group in which the alkyl moiety is linear or branched, a di((C₁-C₆)-hydroxyalkyl)amino group in which each alkyl moiety is linear or branched, a -C(O)-Ra group, an -NH-C(O)-Ra group and a linear or branched (C₁-C₆)alkylene chain substituted by one or more identical or different groups selected from halogen atoms and the groups cyano, nitro, -ORa, -NRaRb, -CO₂Ra, -C(O)Ra, (C₁-C₆)hydroxyalkylamino in which the alkyl moiety is linear or branched, di((C₁-C₆)hydroxyalkyl)amino in which each alkyl moiety is linear or branched, and -C(O)-NHRa, the groups Ra, Rb and T₂ being as defined hereinbefore,
• **X** represents a group selected from a hydrogen atom, a hydroxy group, a linear or branched (C₁-C₆)alkoxy group, a mercapto group and a linear or branched (C₁-C₆)alkyl-thio group,
• **Y** represents a hydrogen atom,
or
**X** and **Y** together with the carbon atom carrying them form a carbonyl group,
• **X**_{**1**} represents a group selected from a hydrogen atom, a hydroxy group, a linear or branched (C₁-C₆)alkoxy group, a mercapto group and a linear or branched (C₁-C₆)alkyl-thio group,
• **Y**_{**1**} represents a hydrogen atom,
or
**X**_{**1**} and **Y**_{**1**} together with the carbon atom carrying them form a carbonyl group,
• **R**_{**4**} and **R**_{**5**}**,** which may be identical or different, each independently of the other represents a group selected from a hydrogen atom, a halogen atom, a hydroxy group, a linear or branched (C₁-C₆)alkoxy group, an aryloxy group, an aryl-(C₁-C₆)alkoxy group in which the alkoxy moiety is linear or branched, a linear or branched (C₁-C₆)alkyl group, an aryl-(C₁-C₆)alkyl group in which the alkyl moiety is linear or branched, an aryl group, an amino group (itself being optionally substituted by one or two identical or different groups selected from linear or branched (C₁-C₆)alkyl, aryl and aryl-(C₁-C₆)alkyl in which the alkyl moiety is linear or branched), an azido group, an -N=NRa group (wherein Ra is as defined hereinbefore), and an -O-C(O)-Rc group wherein Rc represents a linear or branched (C₁-C₆)alkyl group (optionally substituted by one or more groups selected from halogen, hydroxy, amino, linear or branched (C₁-C₆)alkylamino and di-(C₁-C₆)alkyl-amino in which each alkyl moiety is linear or branched), an aryl group, an aryl-(C₁-C₆)-alkyl group in which the alkyl moiety is linear or branched, a cycloalkyl group or a heterocycloalkyl group,
• **R**_{**6**} represents a group of formula -U₁-R₄ wherein U₁ represents a single bond or a methylene group, and R₄ is as defined hereinbefore,
• or **R**_{**4**}**, R**_{**5**} and **R**_{**6**} taken in pairs in adjacent or non-adjacent positions form with the carbon atoms carrying them a ring system having from 3 to 6 ring members, containing one or two oxygen atoms, with the remaining group R₄, R₅ or R₆ that does not belong to the ring system having any one of the definitions of R₄, R₅, or R₆ given hereinbefore,
their isomers, and addition salts thereof with a pharmaceutically acceptable acid or base, provided that the compounds of formula (I) are other than the following compounds:
- 1,11-dichloro-12,13-[1,2-(4-O-methyl-β-D-mannopyranosyl)]-6,7,12,13-tetrahydro-(5*H*)-indolo[2,3-a]pyrrolo[3,4-c]carbazole-5,7-dione,
- 12,13-[1,2-(4-O-methyl-β-D-mannopyranosyl)-6,7,12,13-tetrahydro-(5*H*)-indolo[2,3-a]-pyrrolo[3,4-c]carbazole-5,7-dione,
- 12,13-[1,2-(4-O-methyl-β-D-mannopyranosyl)-6,7,12,13-tetrahydro-(5*H*)-indolo[2,3-a]-pyrrolo[3,4-c]carbazol-5-one, and
- 12,13-[1,2-(4-O-methyl-β-D-mannopyranosyl)-5,6,12,13-tetrahydro-(7*H*)-indolo[2,3-a]-pyrrolo[3,4-c]-carbazol-7-one,
it also being understood that:
- "cycloalkyl" is understood to mean a monocyclic or bicyclic group that is saturated or unsaturated but without aromatic character, having from 3 to 10 carbon atoms, being optionally substituted by one or more identical or different groups selected from halogen, linear or branched (C₁-C₆)alkyl, linear or branched (C₁-C₆)trihaloalkyl, hydroxy, linear or branched (C₁-C₆)alkoxy, and amino optionally substituted by one or two linear or branched (C₁-C₆)alkyl groups,
- "heterocycloalkyl" is understood to mean a cycloalkyl group as defined above having within the ring system one or two identical or different hetero atoms selected from oxygen, nitrogen and sulphur,
- "aryl" is understood to mean a phenyl, naphthyl, dihydronaphthyl, tetrahydronaphthyl, indenyl or indanyl group, each of those groups being optionally substituted by one or more identical or different groups selected from halogen, linear or branched (C₁-C₆)-alkyl, linear or branched (C₁-C₆)trihaloalkyl, hydroxy, linear or branched (C₁-C₆)-alkoxy, and amino optionally substituted by one or two linear or branched (C₁-C₆)alkyl groups.

2. Compounds of formula (I) according to claim 1, **characterised in that** G represents an NR₃ group wherein R₃ is as defined for formula (I), their isomers, and addition salts thereof with a pharmaceutically acceptable acid or base.

3. Compounds of formula (I) according to claim 1, **characterised in that** X and Y together with the carbon atom carrying them form a carbonyl group, and X₁ and Y₁ together with the carbon atom carrying them form a carbonyl group, their isomers, and addition salts thereof with a pharmaceutically acceptable acid or base.

4. Compounds of formula (I) according to claim 1, **characterised in that** they represent compounds of formula (Ibis) : 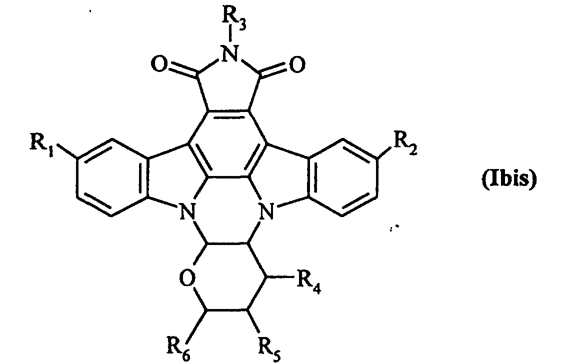 wherein R₁, R₂, R₃, R₄, R₅ and R₆ are as defined for formula (I), their isomers, and addition salts thereof with a pharmaceutically acceptable acid or base.

5. Compounds of formula (I) according to claim 1 or claim 4, **characterised in that** R₁ and R₂, which may be identical or different, represent a group of formula U-V wherein U represents a single bond and V represents a group selected from a halogen atom, a hydrogen atom, a nitro group, a formyl group, a hydroxy group, and a linear or branched (C₁-C₆)alkylene chain substituted by a hydroxy group, their isomers, and addition salts thereof with a pharmaceutically acceptable acid or base.

6. Compounds of formula (I) according to claim 1 or claim 4, **characterised in that** R₁ and R₂ are identical, their isomers and addition salts thereof with a pharmaceutically acceptable acid or base.

7. Compounds of formula (I) according to claim 1 or claim 4, **characterised in that** R₃ represents a hydrogen atom, a hydroxy group or a linear or branched (C₁-C₆)alkylene chain substituted by a group selected from NRaRb and ORa wherein Ra and Rb are as defined for formula (I), their isomers, and addition salts thereof with a pharmaceutically acceptable acid or base.

8. Compounds of formula (I) according to claim 1 or claim 4, **characterised in that** they represent compounds of formula (Iter) : 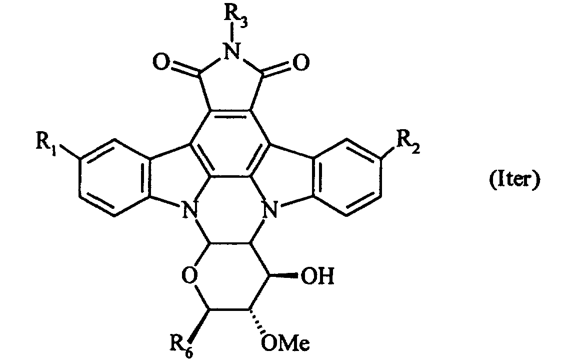 wherein R₁, R₂, R₃ and R₆ are as defined for formula (I), their isomers, and addition salts thereof with a pharmaceutically acceptable acid or base.

9. Compounds of formula (I) which are :
- 3,9-diformyl-12,13-[1,2-(4-*O*-methyl-β-D-mannopyranosyl)]-6,7,12,13-tetrahydro-(5*H*)-indolo[2,3-*a*]pyrrolo[3,4-*c*]carbazole-5,7-dione,
- 3,9-dinitro-12,13-[1,2-(4-*O*-methyl-β-D-mannopyranosyl)]-6,7,12,13-tetrahydro-(5*H*)-indolo[2,3-*a*]pyrrolo[3,4-*c*]carbazole-5,7-dione,
- 3-nitro-12,13-[1,2-(4-*O*-methyl-β-D-mannopyranosyl)]-6,7,12,13-tetrahydro-(5*H*)-indolo[2,3-*a*]pyrrolo[3,4-*c*]carbazole-5,7-dione,
- 9-nitro-12,13-[1,2-(4-*O*-methyl-β-D-mannopyranosyl)]-6,7,12,13-tetrahydro-(5*H*)-indolo[2,3-*a*]pyrrolo[3,4-*c*]carbazole-5,7-dione,
- 6-hydroxy-12,13-[1,2-(4-O-methyl-β-D-mannopyranosyl)]-6,7,12,13-tetrahydro-(5*H*)-indolo[2,3-*a*]pyrrolo[3,4-*c*]carbazole-5,7-dione,
- 3,9-di-(hydroxymethyl)-12,13-[1,2-(4-*O*-methyl-β-D-mannopyranosyl)]-6,7,12,13-tetrahydro-(5*H*)-indolo[2,3-*a*]pyrrolo[3,4-*c*]carbazole-5,7-dione,
- 3,9-dinitro-12,13-[1,2-(3,6-anhydro-4-*O*-methyl-β-D-mannopyranosyl)]-6,7,12,13-tetrahydro-(5*H*)-indolo[2,3-*a*]pyrrolo[3,4-*c*]carbazole-5,7-dione,
- 3,9-dihydroxy-12,13-[1,2-(4-*O*-methyl-β-D-mannopyranosyl)]-6,7,12,13-tetrahydro-(5*H*)-indolo[2,3-*a*]pyrrolo[3,4-*c*]carbazole-5,7-dione,
- 3,9-dibromo-12,13-[1,2-(4-*O*-methyl-β-D-mannopyranosyl)]-6,7,12,13-tetrahydro-(5*H*)-indolo[2,3-*a*]pyrrolo[3,4-*c*]carbazole-5,7-dione, and
- 6-diethylaminoethyl-12,13-[1,2-(4-*O*-methyl-β-D-mannopyranosyl)]-6,7,12,13-tetrahydro-(5*H*)-indolo[2,3-*a*]pyrrolo[3,4-*c*]carbazole-5,7-dione and its hydrochloride, their isomers, and addition salts thereof with a pharmaceutically acceptable acid or base.

10. Process for the preparation of compounds of formula (I), **characterised in that** there is used as starting material a compound of formula (II): 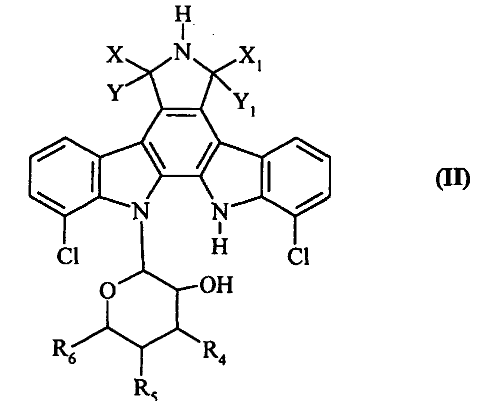 wherein X, Y, X₁, Y₁, R₄, R₅ and R₆ are as defined for formula (I),
which is treated in a basic medium with *para*-toluenesulphonic acid, to yield a compound of formula (III): 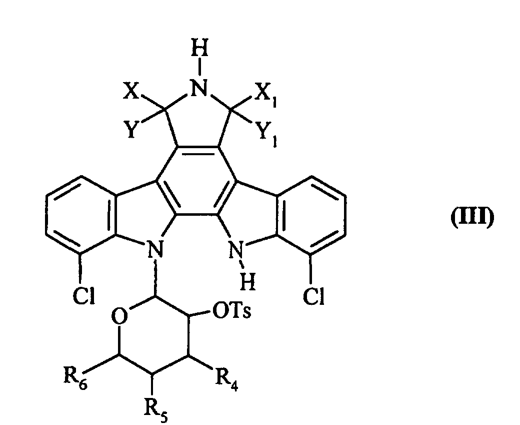 wherein X, Y, X₁, Y₁, R₄, R₅ and R₆ are as defined hereinbefore,
which compound of formula (III) is reacted with sodium azide, to yield principally a compound of formula (I/a), a particular case of the compounds of formula (I) : 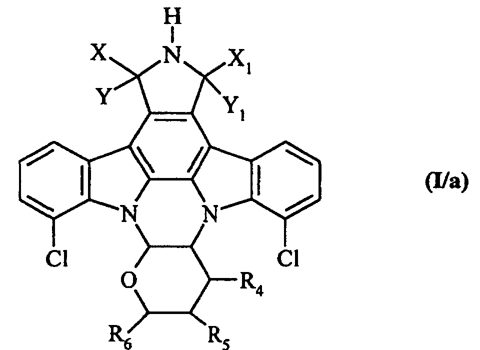 wherein X, Y, X₁, Y₁, R₄, R₅ and R₆ are as defined hereinbefore,
which compound of formula (I/a) is, if desired, subjected to conditions of hydrogenolysis, to yield a compound of formula (I/b), a particular case of the compounds of formula (I) : 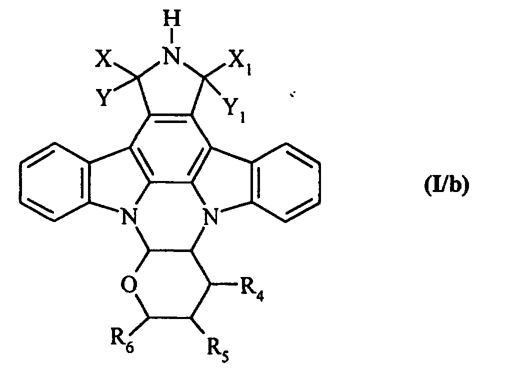 wherein X, Y, X₁, Y₁, R₄, R₅ and R₆ are as defined hereinbefore,
which compound of formula (I/b) is, if desired, treated with aqueous sodium hydroxide solution and then treated with hydrochloric acid, to yield a compound of formula (I/c), a particular case of the compounds of formula (1): 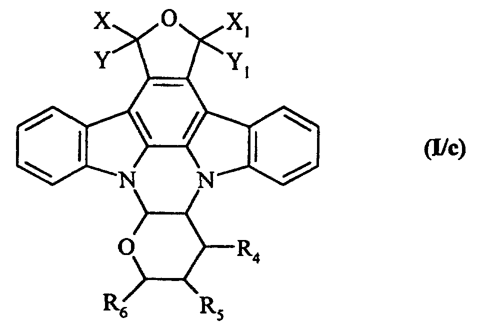 wherein X, Y, X₁, Y₁, R₄, R₅ and R₆ are as defined hereinbefore,
which compound of formula (I/c) is, if desired, subjected to the action of a compound of formula (IV):
^{R}3a ^{- NH}2 (IV)
wherein R₃ₐ has the same definitions as R₃ with the exception of a hydrogen atom, to yield a compound of formula (I/d), a particular case of the compounds of formula (I) : 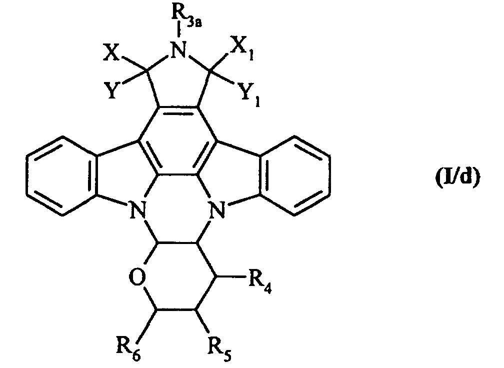 wherein X, Y, X₁, Y₁, R₃ₐ, R₄, R₅ and R₆ are as defined hereinbefore,
the totality of the compounds of formulae (I/b) to (I/d) constituting the compounds of formula (I/e): 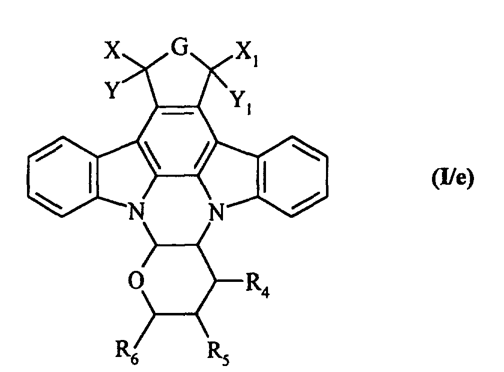 wherein G, X, Y, X₁, Y₁, R₄, R₅ and R₆ are as defined for formula (I), which compound of formula (I/e) is, if desired, subjected to an electrophilic aromatic addition reaction or to a nucleophilic aromatic addition reaction, according to conventional conditions of organic synthesis well known to the person skilled in the art, to yield a compound of formula (I/f), a particular case of the compounds of formula (I) : 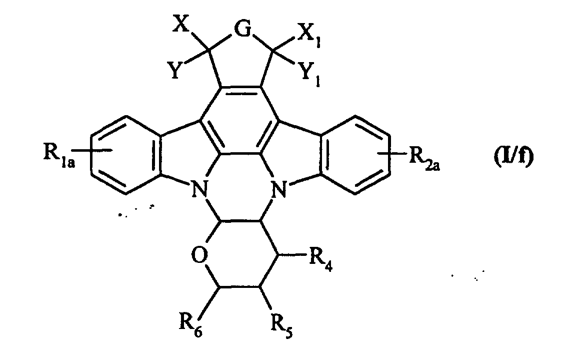 wherein G, X, Y, X₁, Y_{1,} R₄, R₅ and R₆ are as defined hereinbefore, and R₁ₐ and R₂ₐ have the same definitions as R₁ and R₂, respectively, except that R₁ₐ and R₂ₐ cannot simultaneously represent a hydrogen atom,
which compounds of formulae (I/a) to (I/f) constitute the totality of the compounds of formula (I), which are purified, if necessary, according to conventional purification techniques, which may, if desired, be separated into their different isomers according to a conventional separation technique, the substituents R₄, R₅ and R₆ of which are modified according to conventional methods of organic synthesis used in the field of sugar chemistry, and which are converted, if desired, to addition salts with a pharmaceutically acceptable acid or base.

11. Pharmaceutical compositions comprising as active ingredient at least one compound of formula (I) according to any one of claims 1 to 9, on its own or in combination with one or more inert, non-toxic, pharmaceutically acceptable excipients or carriers.

12. Pharmaceutical compositions according to claim 10 for use as anti-tumour agents in the treatment of cancers.

## Patentansprüche

1. Verbindungen der Formel (I): 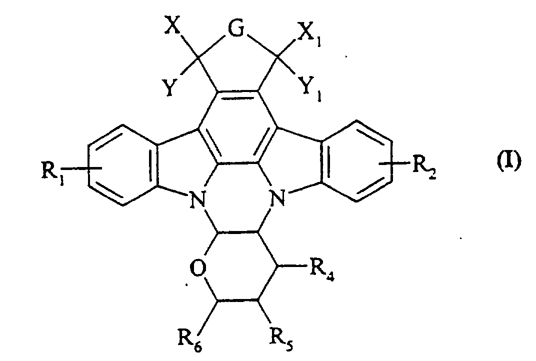 in der:
• **R**_{**1**} und **R**_{**2**}**,** die gleichartig oder verschieden sind, unabhängig voneinander eine Gruppe der Formel U-V darstellen, in der:
* U eine Einfachbindung oder eine geradkettige oder verzweigte (C₁-C₆)-Alkylenkette, die gegebenenfalls durch eine oder mehrere gleichartige oder verschiedene Gruppen ausgewählt aus Halogen und Hydroxy substituiert ist und/oder gegebenenfalls eine oder mehrere Unsättigungen enthält, bedeutet,
* V eine Gruppe ausgewählt aus Wasserstoffatomen, Halogenatomen, Cyanogruppen, Nitrogruppen, Azidogruppen, geradkettigen oder verzweigten (C₁-C₆)-Alkylgruppen, Arylgruppen, geradkettigen oder verzweigten Aryl-(C₁-C₆)-alkylgruppen, Hydroxygruppen, geradkettigen oder verzweigten (C₁-C₆)-Alkoxygruppen. Aryloxygruppen, geradkettigen oder verzweigten Aryl-(C₁-C₆)-alkoxygruppen, Formylgruppen, Carboxygruppen, Aminocarbonylgruppen und Gruppen der Formeln NRaRb, -C(O)-T₁, -C(O)-NRa-T₁, -NRa-C(O)-T₁, -O-C(O)-T₁, -C(O)-O-T₁, -O-T₂-NRaRb, -O-T₂-ORa, -O-T₂-CO₂Ra, -NRa-T₂-NRaRb, -NRa-T₂-ORa, -NRa-T₂-CO₂-Ra und -S(O)ₙ-Ra darstellt,
worin:
→ Ra und Rb, die gleichartig oder verschieden sind, jeweils eine Gruppe ausgewählt aus Wasserstoffatomen, geradkettigen oder verzweigten (C₁-C₆)-Alkylgruppen, Arylgruppen und geradkettigen oder verzweigten (C1-C₆)-Arylalkylgruppen darstellen,
oder
Ra+Rb gemeinsam mit dem sie tragenden Stickstoffatom einen monocyclischen Heterocyclus mit 5 bis 7 Kettengliedern bilden, der gegebenenfalls im Rahmen des Ringsystems ein zweites Heteroatom ausgewählt aus Sauerstoff und Stickstoff enthält und gegebenenfalls durch eine Gruppe ausgewählt aus geradkettigem oder verzweigtem (C₁-C₆)-Alkyl, Hydroxy, geradkettigem oder verzweigtem (C₁-C₆)-Alkoxy, Amino, geradkettigem oder verzweigtem (C₁-C₆)-Alkylamino und geradkettigem oder verzweigtem (C₁-C₆)-Dialkylamino substituiert ist,
→ T₁ eine Gruppe ausgewählt aus geradkettigem oder verzweigtem (C₁-C₆)-Alkyl, Aryl, geradkettigem oder verzweigtem Aryl-(C₁-C₆)-alkyl und einer geradkettigen oder verzweigten (C₁-C₆)-Alkylenkette, die durch eine Gruppe ausgewählt aus -ORa, -NRaRb, -CO₂Ra, -C(O)Ra und -C(O)NRaRb substituiert ist, in denen Ra und Rb die oben angegebenen Bedeutungen besitzen, darstellt,
→ T₂ eine geradkettige oder verzweigte (C₁-C₆)-Alkylenkette darstellt und
→ n eine ganze Zahl mit einem Wert zwischen 0 und 2 einschließlich bedeutet,
• **G** ein Sauerstoffatom oder eine Gruppe NR₃ darstellt, in der R₃ eine Gruppe ausgewählt aus Wasserstoffatomen, geradkettigen oder verzweigten (C₁-C₆)-Alkylgruppen, Arylgruppen, geradkettigen oder verzweigten Aryl-(C₁-C₆)-alkylgruppen, Cycloalkylgruppen, geradkettigen oder verzweigten Cycloalkyl-(C₁-C₆)-alkylgruppen, Gruppen der Formeln -ORa, -NRaRb, -O-T₂-NRaRb, -NRa-T₂-NRaRb, geradkettigen oder verzweigten (C₁-C₆)-Hydroxyalkylaminogruppen, geradkettigen oder verzweigten Di-(C₁-C₆)-(hydroxyalkyl)-aminogruppen, Gruppen der Formeln -C(O)-Ra, -NH-C(O)-Ra und geradkettigen oder verzweigten (C₁-C₆)-Alkylenketten, die durch eine oder mehrere gleichartige oder verschiedenartige Gruppen ausgewählt aus Halogenatomen, Cyanogruppen, Nitrogruppen, Gruppen der Formeln -ORa, -NRaRb, -CO₂Ra, -C(O)Ra, geradkettigen oder verzweigten (C₁-C₆)-Hydroxyalkylaminogruppen, geradkettigen oder verzweigten Di-(C₁-C₆)-(hydroxyalkyl)-aminogruppen und Gruppen der Formel -C(O)-NHRa, worin Ra, Rb und T₂ die oben angegebenen Bedeutungen besitzen, substituiert sind, bedeutet,
• **X** eine Gruppe ausgewählt aus Wasserstoffatomen, geradkettigen oder verzweigten (C₁-C₆)-Alkoxygruppen. Mercaptogruppen und geradkettigen oder verzweigten (C₁-C₆)-Alkylthiogruppen darstellt,
• **Y** ein Wasserstoffatom darstellt.
oder
**X** und **Y** gemeinsam mit dem sie tragenden Kohlenstoffatom eine Carbonylgruppe bilden,
• **X**_{**1**} eine Gruppe ausgewählt aus Wasserstoffatomen, Hydroxygruppen, geradkettigen oder verzweigten (C₁-C₆)-Alkoxygruppen, Mercaptogruppen und geradkettigen oder verzweigten (C₁-C₆)-Alkylthiogruppen darstellt,
• **Y**_{**1**} ein Wasserstoffatom darstellt
oder
**X**_{**1**} und **Y**_{**1**} gemeinsam mit dem sie tragenden Kohlenstoffatom eine Carbonylgruppe bilden,
• **R**_{**4**} und **R**_{**5**}**,** die gleichartig oder verschieden sind, unabhängig voneinander jeweils eine Gruppe ausgewählt aus Wasserstoffatomen. Halogenatomen, Hydroxygruppen, geradkettigen oder verzweigten (C₁-C₆)-Alkoxygruppen, Aryloxygruppen, geradkettigen oder verzweigten Aryl-(C₁-C₆)-alkoxygruppen, geradkettigen oder verzweigten (C₁-C₆)-Alkylgruppen, geradkettigen oder verzweigten Aryl-(C₁-C₆)-alkylgruppen, Arylgruppen. Aminogruppen (die ihrerseits gegebenenfalls durch eine oder zwei gleichartige oder verschiedenartige Gruppen ausgewählt aus geradkettigem oder verzweigtem (C₁-C₆)-Alkyl, Aryl und geradkettigem oder verzweigtem Aryl-(C₁-C₆)-alkyl substituiert sind), Azidogruppen, Gruppen der Formeln -N=NRa (worin Ra die oben angegebenen Bedeutungen besitzt) und -O-C(O)-Rc, in der Rc eine geradkettige oder verzweigte (C₁-C₆)-Alkylgrupppe (die gegebenenfalls durch eine oder mehrere Gruppen ausgewählt aus Halogen, Hydroxy, Amino, geradkettigem oder verzweigtem (C₁-C₆)-Alkylamino und geradkettigem oder verzweigtem (C₁-C₆)-Dialkylamino substituiert ist), Arylgruppe, geradkettige oder verzweigte Aryl-(C₁-C₆)-alkylgruppe, Cycloalkylgruppe oder Heterocycloalkylgruppe bedeutet, darstellen,
• **R**_{**6**} eine Gruppe der Formel -U₁-R₄ darstellt, worin U₁ eine Einfachbindung oder eine Methylengruppe bedeutet und R₄ die oben angegebenen Bedeutungen besitzt,
• oder **R**_{**4**}**, R**_{**5**} und **R**_{**6**} jeweils zu zweit in benachbarten oder nicht benachbarten Positionen zusammen mit den sie tragenden Kohlenstoffatomen ein cyclisches System mit 3 bis 6 Kettengliedern bilden, welches ein oder zwei Sauerstoffatome enthält, wobei die verbleibende Gruppe R₄, R₅ oder R₆ nicht dem cyclischen System angehört und demzufolge irgendeine oben angegebene Definition von R₄, R₅ oder R₆ annimmt,
deren Isomere sowie deren Additionssalze mit einer pharmazeutisch annehmbaren Säure oder Base,
mit der Maßgabe, daß die Verbindungen der Formel (I) von den folgenden Verbindungen verschieden sind:
- 1,11-Dichlor-12,13-[1,2-(4-O-methyl-β-D-mannopyranosyl)]-6,7,12,13-tetrahydro-(5H)-indolo(2,3-alpyrrolo(3,4-c]carbazol-5,7-dion,
- 12,13-[1,2-(4-O-Methyl-β-D-mannopyranosyl)-6,7,12,13-tetrahydro(5*H*)-indolo[2,3-a]pyrrolo[3,4-c]carbazol-5,7-dion,
- 12,13-[1,2-(4-O-Methyl-β-D-mannopyranosyl)-6, 7,12,13-tetrahydro(5*H*)-indolo[2,3-a]pyrrolo(3,4-c]carbazol-5-on und
- 12,13-[1,2-(4-O-Methyl-β-D-mannopyranosyl)-5,6,12,13-tetrahydro(7*H*)-indolo-[2,3-a]pyrrolo(3,4-c]carbazol-7-on,
und weiter mit der Maßgabe, daß:
- unter Cycloalkyl eine monocyclische oder bicyclische, gesättigte oder ungesättigte Gruppe ohne aromatischen Charakter zu verstehen ist, die 3 bis 10 Kohlenstoffatome aufweist und gegebenenfalls durch eine oder mehrere gleichartige oder verschiedene Gruppen ausgewählt aus Halogen, geradkettigem oder verzweigtem (C₁-C₆)-Alkyl, geradkettigem oder verzweigtem (C₁-C₆)-Trihalogenalkyl, Hydroxy, geradkettigem oder verzweigtem (C₁-C₆)-Alkoxy und gegebenenfalls durch eine oder zwei geradkettige oder verzweigte (C₁-C₆)-Alkylgruppen substituiertem Amino substituiert ist,
- unter Heterocycloalkyl eine Cycloalkylgruppe zu verstehen ist, wie sie oben definiert worden ist, die in dem cyclischen System ein oder zwei gleichartige oder verschiedene Heteroatome ausgewählt aus Sauerstoff, Stickstoff und Schwefel umfaßt,
- unter Aryl eine Phenyl-, Naphthyl-, Dihydronaphthyl-, Tetrahydronaphthyl-, Indenyl- oder Indanylgruppe zu verstehen ist, wobei jede dieser Gruppen gegebenenfalls durch eine oder mehrere gleichartige oder verschiedene Gruppen ausgewählt aus Halogen, geradkettigem oder verzweigtem (C₁-C₆)-Alkyl, geradkettigem oder verzweigtem (C₁-C₆)-Trihalogenalkyl, Hydroxy, geradkettigem oder verzweigtem (C₁-C₆)-Alkoxy und gegebenenfalls durch eine oder zwei geradkettige oder verzweigte (C₁-C₆)-Alkylgruppen substituiertem Amino substituiert ist.

2. Verbindungen der Formel (I) nach Anspruch 1. **dadurch gekennzeichnet, daß** G eine Gruppe NR₃ darstellt, in der R₃ die bezüglich der Formel (I) angegebenen Bedeutungen besitzt, deren Isomere sowie deren Additionssalze mit einer pharmazeutisch annehmbaren Säure oder Base.

3. Verbindungen der Formel (I) nach Anspruch 1, **dadurch gekennzeichnet, daß** X und Y gemeinsam mit dem sie tragenden Kohlenstoffatom eine Carbonylgruppe bilden und X₁ und Y₁ gemeinsam mit dem sie tragenden Kohlenstoffatom eine Carbonylgruppe bilden, deren Isomere sowie deren Additionssalze mit einer pharmazeutisch annehmbaren Säure oder Base.

4. Verbindungen der Formel (I) nach Anspruch 1, **dadurch gekennzeichnet, daß** sie der Formel (Ibis) entsprechen: 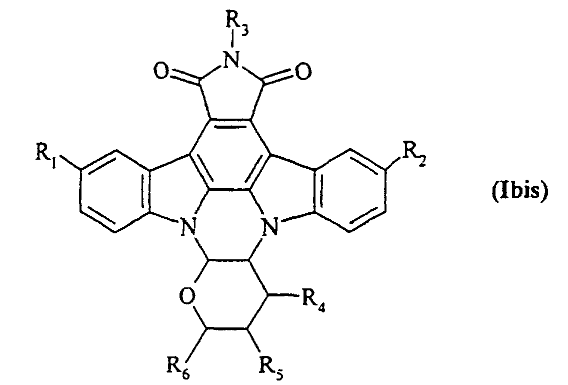 in der R₁, R₂, R₃, R₄, R₅ und R₆ die bezüglich der Formel (I) angegebenen Bedeutungen besitzen, deren Isomere sowie deren Additionssalze mit einer pharmazeutisch annehmbaren Säure oder Base.

5. Verbindungen der Formel (I) nach Anspruch 1 oder 4, **dadurch gekennzeichnet, daß** R₁ und R₂, die gleichartig oder verschieden sind, eine Gruppe der Formel U-V darstellen, worin U eine Einfachbindung und V eine Gruppe ausgewählt aus Halogenatomen, Wasserstoffatomen, Nitrogruppen, Formylgruppen, Hydroxygruppen und geradkettigen oder verzweigten (C₁-C₆)-Alkylenketten, die durch eine Hydroxygruppe substituiert sind, bedeuten, deren Isomere sowie deren Additionssalze mit einer pharmazeutisch annehmbaren Säure oder Base.

6. Verbindungen der Formel (I) nach Anspruch 1 oder 4, **dadurch gekennzeichnet, daß** R₁ und R₂ identisch sind, deren Isomere sowie deren Additionssalze mit einer pharmazeutisch annehmbaren Säure oder Base.

7. Verbindungen der Formel (I) nach Anspruch 1 oder 4, **dadurch gekennzeichnet, daß** R₃ ein Wasserstoffatom, eine Hydroxygruppe oder eine geradkettige oder verzweigte (C₁-C₆)-Alkylenkette, die durch eine Gruppe ausgewählt aus NRaRb und ORa substituiert ist, in der Ra und Rb die bezüglich der Formel (I) angegebenen Bedeutungen besitzen, darstellt, deren Isomere sowie deren Additionssalze mit einer pharmazeutisch annehmbaren Säure oder Base.

8. Verbindungen der Formel (I) nach Anspruch 1 oder 4, **dadurch gekennzeichnet. daß** sie der Formel (Iter) entsprechen: 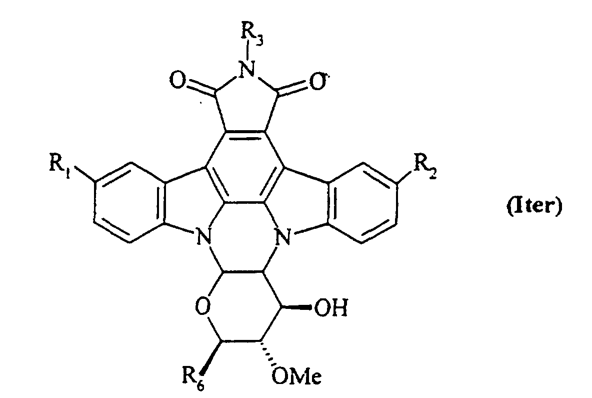 in der R₁, R₂, R₃ und R₆ die bezüglich der Formel (I) angegebenen Bedeutungen besitzen, deren Isomere sowie deren Additionssalze mit einer pharmazeutisch annehmbaren Säure oder Base.

9. Verbindungen der Formel (I), nämlich:
- 3,9-Diformyl-12,13-[1,2-(4-*O*-methyl-β-D-mannopyranosyl)]-6,7,12,13-tetrahydro(5*H*)-indolo[2,3-*a*]pyrrolo[3,4-*c*]carbazol-5,7-dion.
- 3,9-Dinitro-12,13-(1,2-(4-*O*-methyl-β-D-mannopyranosyl)]-6,7,12,13-tetrahydro(5*H*)-indolo[2,3-*a*]pyrrolo[3,4-*c*]carbazol-5,7-dion,
- 3-Nitro-12,13-[1,2-(4-*O*-methyl-β-D-mannopyranosyl)]-6,7,12,13-tetrahydro-(5*H*)-indolo[2,3-*a*]pyrrolo[3,4-*c*]carbazol-5,7-dion,
- 9-Nitro-12,13-[1,2-(4-*O*-methyl-β-D-mannopyranosyl)]-6,7,12,13-tetrahydro-(5*H*)-indolo[2,3-*a*]pyrrolo[3,4-*c*]carbazol-5,7-dion,
- 6-Hydroxy-12,13-(1,2-(4-*O*-methyl-β-D-mannopyranosyl)]-6,7,12,13-tetrahydro-(5*H*)-indolo[2,3-*a*]pyrrolo[3,4-*c*]carbazol-5,7-dion,
- 3,9-Di-(hydroxymethyl)-12,13-[1,2-(4-*O*-methyl-β-D-mannopyranosyl)]-6,7,12,13-tetrahydro(5*H*)-indolo[2,3-*a*]pyrrolo[3,4-*c*]carbazol-5,7-dion,
- 3,9-Dinitro-12,13-[1,2-(3,6-anhydro-4-*O*-methyl-β-D-mannopyranosyl)]-6,7,12,13-tetrahydro(5*H*)-indolo[2,3-*a*]pyrrolo[3,4-*c*]carbazol-5,7-dion,
- 3,9-Dihydroxy-12,13-[1,2-(4-*O*-methyl-β-D-mannopyranosyl)]-6,7,12,13-tetrahydro(5*H*)-indolo[2,3-*a*]pyrrolo[3,4-*c*]carbazol-5,7-dion,
- 3,9-Dibrom-12,13-[1,2-(4-*O*-methyl-β-D-mannopyranosyl)]-6,7,12,13-tetrahydro(5*H*)-indolo[2,3-*a*]pyrrolo[3,4-*c*]carbazol-5,7-dion,
- und 6-Diethylaminoethyl-12,13-[1,2-(4-*O*-methyl-β-D-mannopyranosyl)]-6,7,12,13-tetrahydro(5*H*)-indolo[2,3-*a*]pyrrolo[3,4-*c*]carbazol-5,7-dion und dessen Hydrochlorid,
deren Isomeren sowie deren Additionssalze mit einer pharmazeutisch annehmbaren Säure oder Base.

10. Verfahren zur Herstellung der Verbindungen der Formel (I), **dadurch gekennzeichnet, daß** man als Ausgangsprodukt eine Verbindung der Formel (II) verwendet: 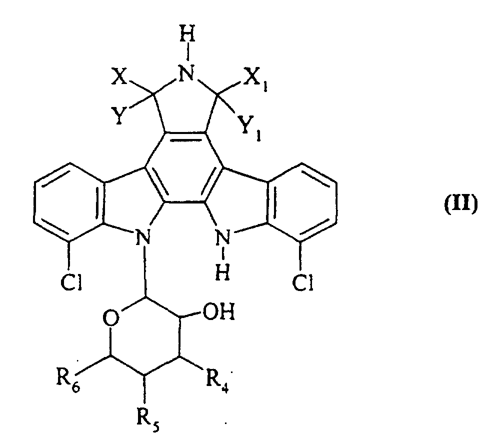 in der X, Y, X₁, Y₁, R₄, R₅ und R₆ die bezüglich der Formel (I) angegebenen Bedeutungen besitzen,
welche man in basischem Medium und in Gegenwart von p-Toluolsulfonsäure behandelt zur Bildung der Verbindung der Formel (III): 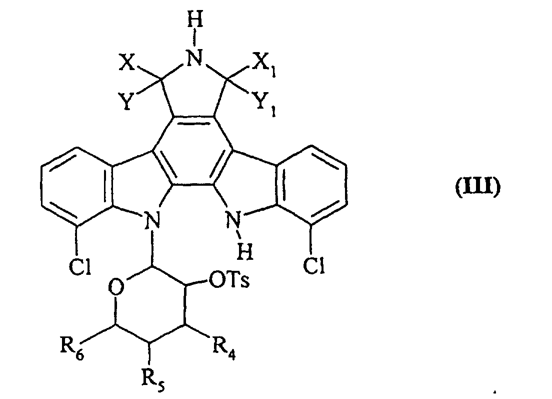 in der X, Y, X₁, Y₁, R₄, R₅ und R₆ die oben angegebenen Bedeutungen besitzen, welche Verbindung des Formel (III) mit Natriumazid umgesetzt wird zur überwiegenden Bildung der Verbindung der Formel (I/a), einem Sonderfall der Verbindungen der Formel (I) : 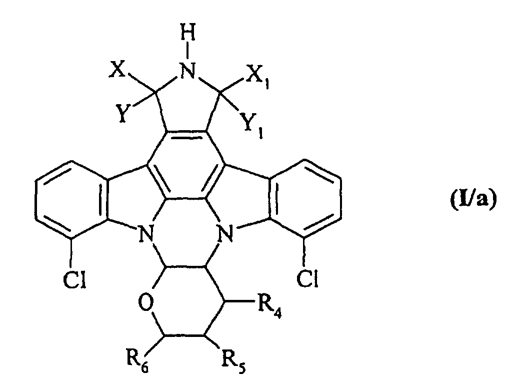 in der X, Y, X₁, Y₁, R₄, R₅ und R₆ die oben angegebenen Bedeutungen besitzen, welche Verbindung der Formel (I/a) gegebenenfalls den Bedingungen der Hydrogenolyse unterworfen wird zur Bildung der Verbindung der Formel (I/b), einem Sonderfall der Verbindungen der Formel (I): 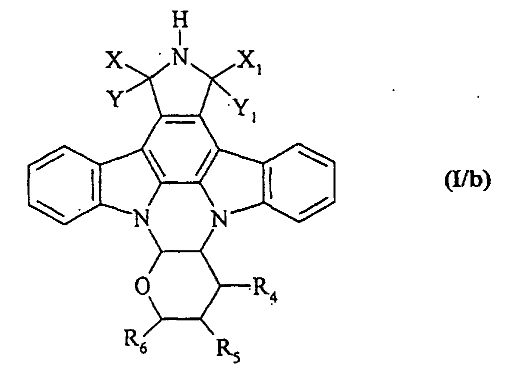 in der X, Y, X₁, Y₁, R₄, R₅ und R₆ die oben angegebenen Bedeutungen besitzen. welche Verbindung der Formel (I/b) man gegebenenfalls mit wäßrigem Natriumhydroxid behandelt und dann in Gegenwart von Chlorwasserstoffsäure bringt zur Bildung der Verbindung der Formel (I/c), einem Sonderfall der Verbindungen der Formel (I): 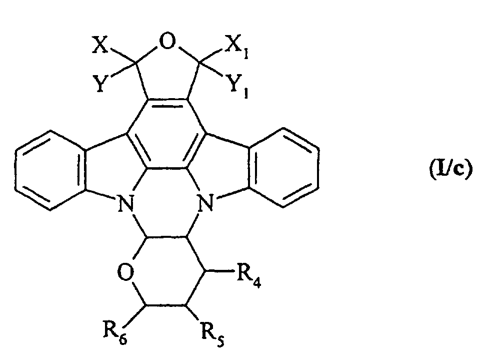 in der X, Y, X₁, Y₁, R₄, R₅ und R₆ die oben angegebenen Bedeutungen besitzen, welche Verbindung der Formel (I/c) gegebenenfalls der Einwirkung einer Verbindung der Formel (IV) unterworfen wird:
R₃ₐ - NH₂ (IV)
in der R₃ₐ die gleichen Bedeutungen besitzt wie R₃ mit Ausnahme der Definition des Wasserstoffatoms,
zur Bildung der Verbindung der Formel (I/d), einem Sonderfall der Verbindungen der Formel (I): 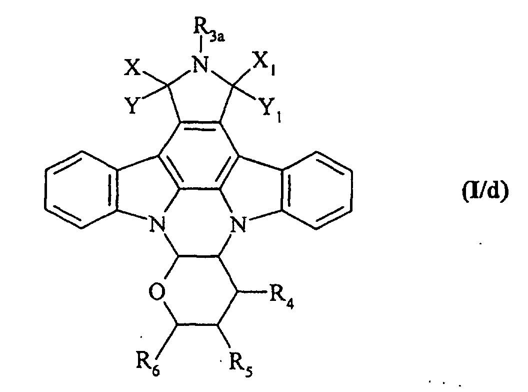 in der X, Y, X₁, Y₁, R₃ₐ, R₄, R₅ und R₆ die oben angegebenen Bedeutungen besitzen,
wobei die Gesamtheit der Verbindungen der Formel (I/b) bis (I/d) die Verbindungen der Formel (I/e) bilden: 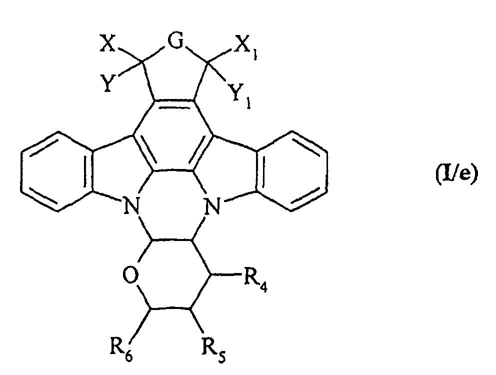 in der G, X, Y, X₁, Y₁, R₄, R₅ und R₆ die bezüglich der Formel (I) angegebenen Bedeutungen besitzen,
welche Verbindung der Formel (I/e) gegebenenfalls einer aromatischen elektrophilen Additionsreaktion oder einer aromatischen nucleophilen Additionsreaktion unter Anwendung klassischer Bedingungen der organischen Synthese, die dem Fachmann wohlbekannt sind, unterworfen wird zur Bildung der Verbindung der Formel (I/f), einem Sonderfall der Verbindungen der Formel (I): 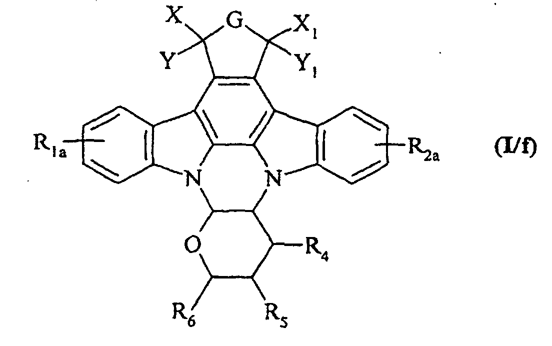 in der G, X, Y, X₁, Y₁, R₄, R₅ und R₆ die oben angegebenen Bedeutungen besitzen und R₁ₐ und R₂ₐ die gleichen Bedeutungen wie R₁ bzw. R₂ besitzen mit dem Unterschied, daß R₁ₐ und R₂ₐ nicht gleichzeitig ein Wasserstoffatom bedeuten können,
wobei die Verbindungen der Formeln (I/a) bis (I/f) die Gesamtheit der Verbindungen der Formel (I) bilden, welche man gegebenenfalls mit Hilfe klassischer Reinigungsmethoden reinigt, welche gewünschtenfalls mit Hilfe einer klassischen Trennmethode in ihre verschiedenen Isomeren getrennt werden können, bei denen man die Substituenten R₄, R₅ und R₆ mit Hilfe klassischer Methoden der organischen Synthese, die in dem Bereich der Zuckerchemie angewandt werden, modifiziert und welche man gewünschtenfalls in ihre Additionssalze mit einer pharmazeutisch annehmbaren Säure oder Base umwandelt.

11. Pharmazeutische Zubereitungen enthaltend als Wirkstoff mindestens eine Verbindung der Formel (I) nach irgendeinem der Ansprüche 1 bis 9 allein oder in Kombination mit einem oder mehreren inerten, nichttoxischen pharmazeutisch annehmbaren Trägermaterialien oder Bindemitteln.

12. Pharmazeutische Zubereitungen nach Anspruch 10, nützlich als antitumorale Mittel bei der Behandlung von Krebs.
